(19) Europäisches Patentamt / European Patent Office / Office européen des brevets

(11) **EP 4 226 852 B1**

(12) **EUROPEAN PATENT SPECIFICATION**

(45) Date of publication and mention
of the grant of the patent:
**05.03.2025 Bulletin 2025/10**

(21) Application number: **21877337.2**

(22) Date of filing: **16.09.2021**

(51) International Patent Classification (IPC):
*A61B 5/02* *(2006.01)*   *A61B 5/026* *(2006.01)*
*A61B 5/0285* *(2006.01)*   *A61B 5/00* *(2006.01)*
*A61B 5/024* *(2006.01)*

(52) Cooperative Patent Classification (CPC):
**A61B 5/0261; A61B 5/0285; A61B 5/6814;
A61B 5/721; A61B 5/7246; A61B 5/7264;**
A61B 5/02416

(86) International application number:
**PCT/JP2021/034094**

(87) International publication number:
**WO 2022/075036 (14.04.2022 Gazette 2022/15)**

(54) **BIOLOGICAL INFORMATION ACQUISITION DEVICE AND BIOLOGICAL INFORMATION ACQUISITION METHOD**

VORRICHTUNG ZUR ERFASSUNG BIOLOGISCHER INFORMATIONEN UND VERFAHREN ZUR ERFASSUNG BIOLOGISCHER INFORMATIONEN

DISPOSITIF D'ACQUISITION D'INFORMATIONS BIOLOGIQUES ET PROCÉDÉ D'ACQUISITION D'INFORMATIONS BIOLOGIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.10.2020 JP 2020168305**

(43) Date of publication of application:
**16.08.2023 Bulletin 2023/33**

(73) Proprietor: **Sony Group Corporation
Tokyo 108-0075 (JP)**

(72) Inventors:
• **KITAKAMI, Yukinojo
Tokyo 108-0075 (JP)**

• **ISHIKAWA, Takanori
Tokyo 108-0075 (JP)**

(74) Representative: **D Young & Co LLP
3 Noble Street
London EC2V 7BQ (GB)**

(56) References cited:
WO-A1-2015/129843   WO-A1-2017/199597
JP-A- 2011 092 236   JP-A- 2012 130 391
JP-A- 2012 130 391   US-A1- 2011 169 978
US-A1- 2011 245 628   US-A1- 2018 000 424
US-A1- 2020 037 902

**Description**

[Technical Field]

The present technique relates to a biological information obtainment device and a biological information obtainment method.

[Background Art]

Thus far, hemodynamic signals detected from organisms have been used to determine the health states and/or psychological states of humans, animals, and the like. This hemodynamic signal is a composite of component signals produced by multiple factors, such as a heartbeat component, a vasomotion component, a pseudo-blood flow component produced by motion of the organism, a blood pressure component, and the like, for example. There is thus a problem in that measuring a health state and/or a psychological state based on a composite hemodynamic signal results in a drop in accuracy.

To solve this problem, a technique is used which extracts a desired component signal from a composite hemodynamic signal. For example, PTL 1 discloses "a data processing device for extracting a desired vital signal including a physiological information component pertaining to a subject from sensor data including time-dependent first sensor data, which includes the physiological information component and at least one motion artifact component, and time-dependent second sensor data, which indicates a position, velocity, or acceleration of a sensing region as a time function in at least one spatial dimension, the data processing device comprising: a decomposition unit that receives the second sensor data, decomposes sensor data decomposed from the second sensor data into at least two components, and based on the decomposed second sensor data, provides at least two different sets of body motion reference data over at least two different body motion reference data channels; and an artifact removal unit that receives the first sensor data and the at least two different body motion reference data channels, and determines the vital signal formed from a combination of the first sensor data and the body motion reference data constituted by at least two of the body motion reference data channels, the artifact removal unit providing the vital signal as the output of the artifact removal unit".

[Citation List]

[Patent Literature]

[PTL 1]
Japanese Translation of PCT Application No. 2017-519548
Other prior art is provided in JP2012-130391A and US2011/169978A.

[Summary]

[Technical Problem]

However, there is a problem in that features in the component signals extracted from a hemodynamic signal cannot be obtained accurately, which makes it impossible to accurately identify those component signals.
Accordingly, a main object of the present technique is to provide a biological information obtainment device and a biological information obtainment method which accurately identify hemodynamic elements included in a hemodynamic signal.

[Solution to Problem]

The present technique provides a biological information obtainment device according to claim 1.
The element may include a heartbeat component, a vasomotion component, and/or a pseudo-blood flow component.
The signal decomposition unit may use empirical mode decomposition.
The periodicity may include autocorrelation.
An autocorrelation $R(\tau)$ may be calculated according to Formula (1) below using a value $v(i)$ of the component signal at time i, a delay time $\tau$, and a number N of sampling data contained in the component signal. [Math. 1]

$$R(\tau) = \frac{1}{N} \sum_{i=1}^{N} v(i) \cdot v(i + \tau) \quad \cdots \quad (1)$$

The identification unit may identify a component signal for which the evaluation value E of the correlation is highest as a heartbeat component, and may identify a component signal for which the evaluation value E of the correlation is lowest as a vasomotion component.

The biological information obtainment device may further include a detection unit that detects the hemodynamic signal.

The detection unit may include: a light emitting unit that irradiates the organism with light; and a light receiving unit that detects light produced as a result of irradiating the organism with the light.

The detection unit may include at least two of the light receiving units.

The biological information obtainment device may further include: a detection unit, attached to a head, that detects the hemodynamic signal; a position change amount measurement unit that measures an amount of change in a position of the detection unit; a correction information generation unit that generates correction information for a blood flow velocity based on the amount of change in the position; and a correction unit that, using the correction information, corrects a blood flow velocity included in the hemodynamic signal detected by the detection unit.

The amount of change may be an amount of change in a tilt of the detection unit or an amount of change in a height of the detection unit.

The signal decomposition unit may decompose the hemodynamic signal including the blood flow velocity corrected by the correction unit into a plurality of component signals, and the identification unit may identify the hemodynamic element based on a correlation between the component signals and the correction information.

[Brief Description of Drawings]

[Fig. 1]

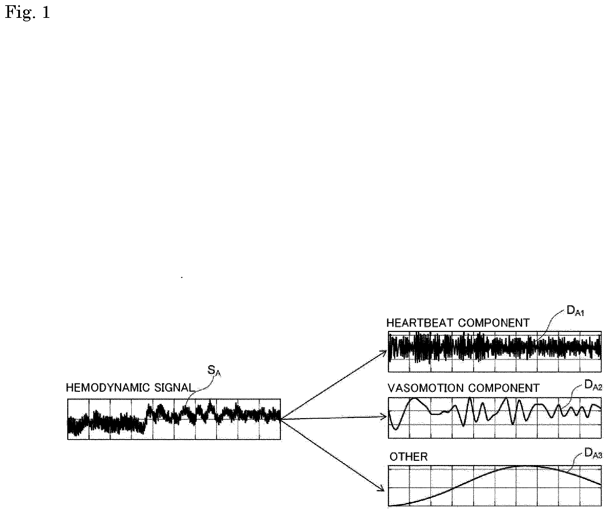

Fig. 1 is a graph showing an example of a hemodynamic signal obtained by a biological information obtainment device 100 according to one embodiment of the present technique.
[Fig. 2]

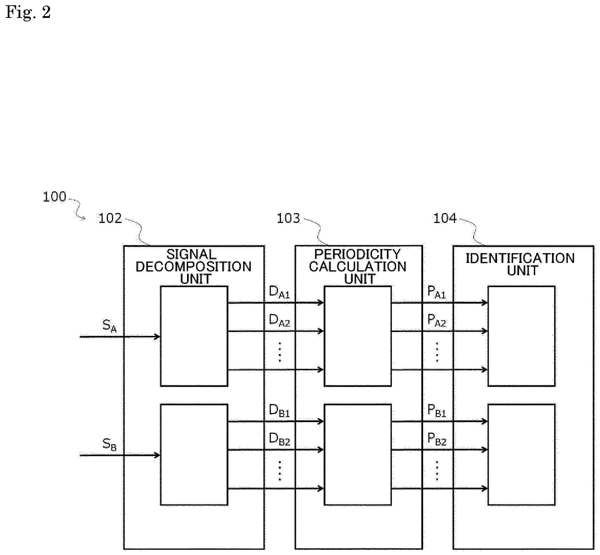

Fig. 2 is a block diagram illustrating the configuration of a biological information obtainment device 100 according to a first embodiment of the present technique.
[Fig. 3]

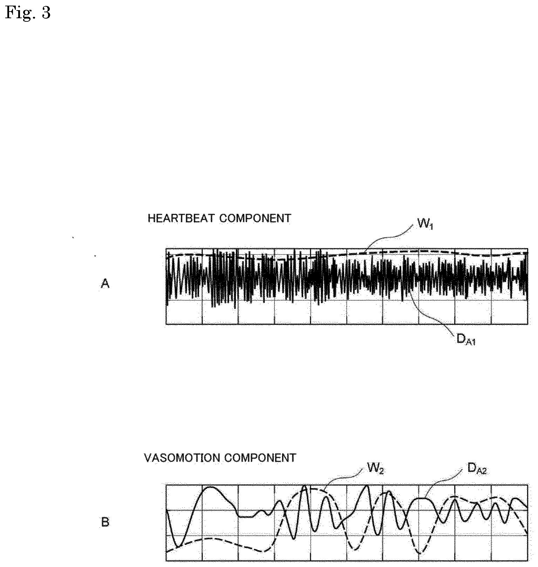

Fig. 3 is a graph showing examples of component signals and autocorrelation obtained by the biological information obtainment device 100 according to one embodiment of the present technique.
[Fig. 4]

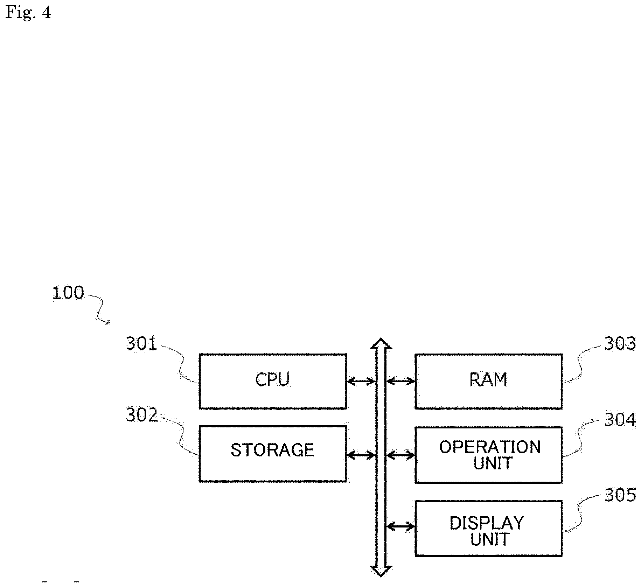

Fig. 4 is a block diagram illustrating the hardware configuration of the biological information obtainment device 100 according to one embodiment of the present technique.
[Fig. 5]

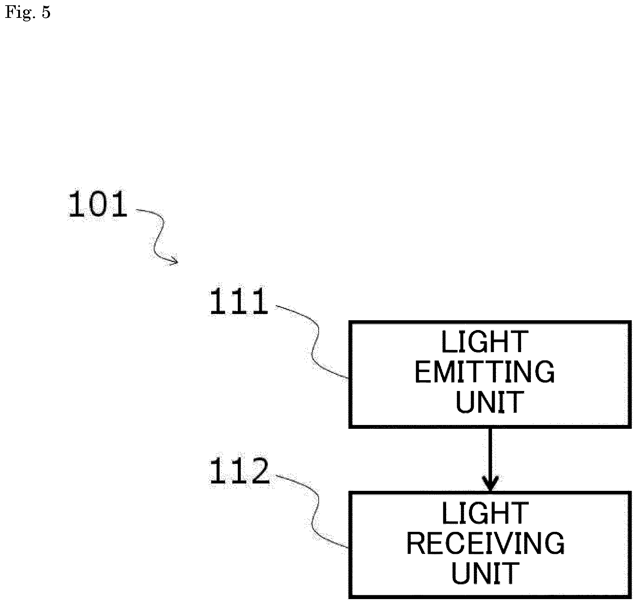

Fig. 5 is a block diagram illustrating the configuration of a detection unit 101 according to one embodiment of the present technique.
[Fig. 6]

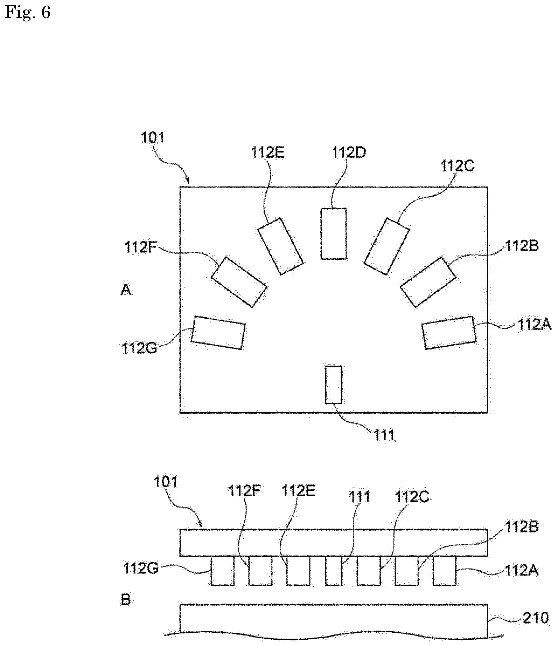

Fig. 6 is a schematic diagram illustrating the detection unit 101 according to one embodiment of the present technique.
[Fig. 7]

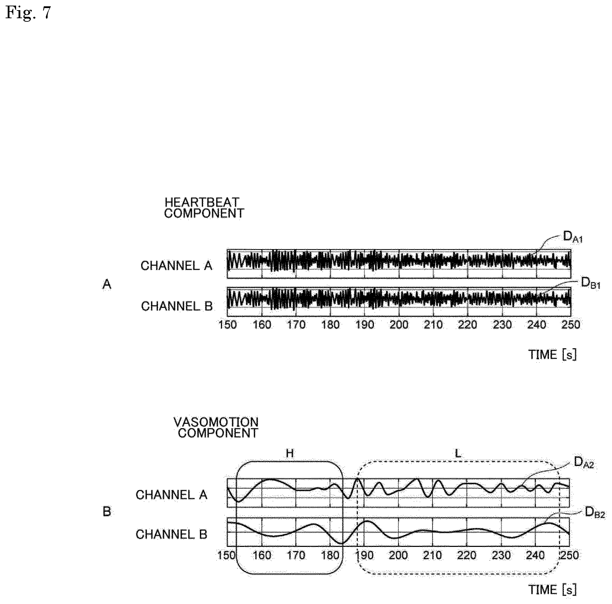

Fig. 7 is a graph showing examples of component signals obtained by the biological information obtainment device 100 according to one embodiment of the present technique.
[Fig. 8]

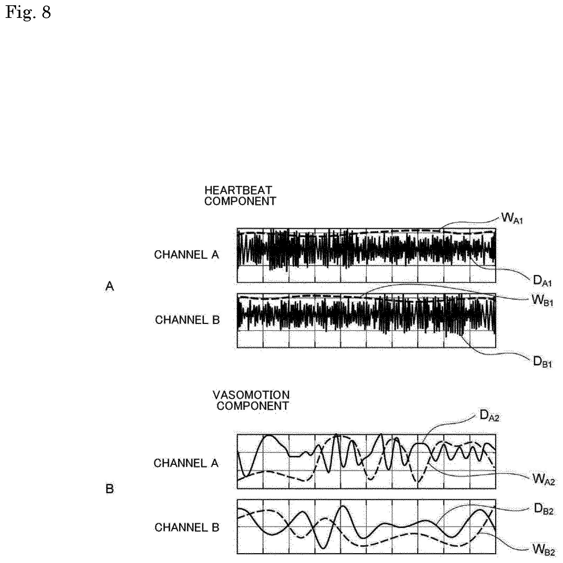

Fig. 8 is a graph showing examples of component signals and autocorrelation obtained by the biological information obtainment device 100 according to one embodiment of the present technique.
[Fig. 9]

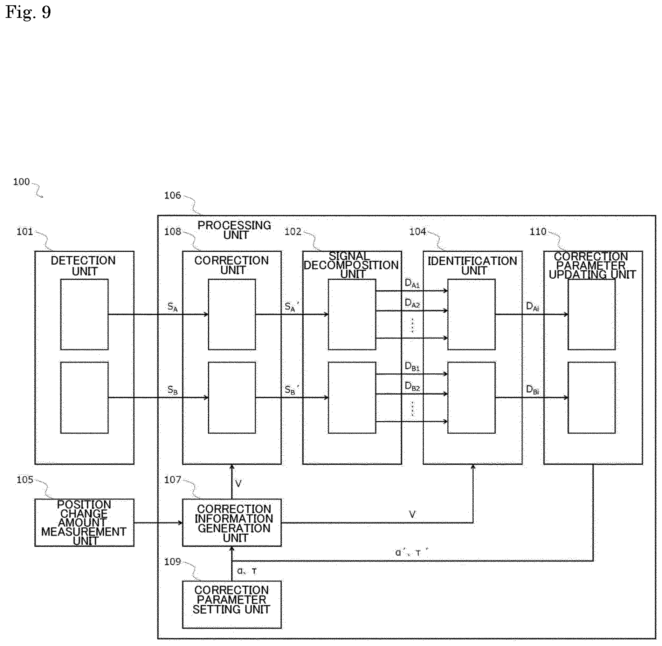

Fig. 9 is a block diagram illustrating the configuration of a biological information obtainment device 100 according to a third embodiment of the present technique.

[Fig. 10]

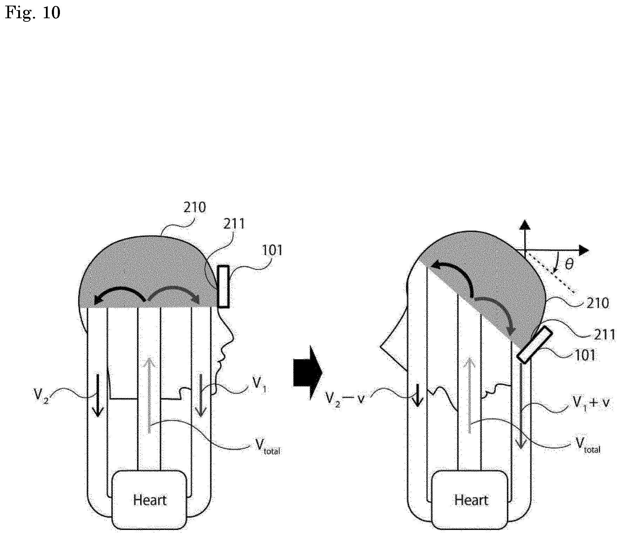

Fig. 10 is a model diagram illustrating changes in blood flow velocity caused by changes in the position of the head.
[Fig. 11]

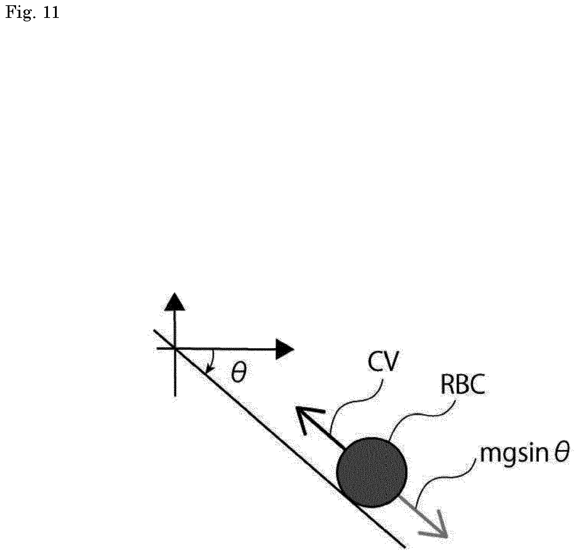

Fig. 11 is a model diagram illustrating resistance force and gravity acting on red blood cells.
[Fig. 12]

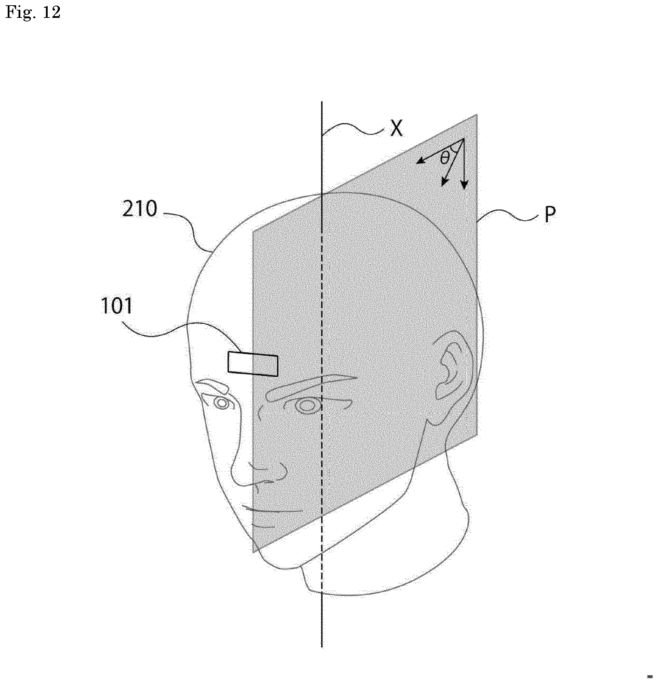

Fig. 12 is a diagram illustrating changes in the tilt of the head.
[Fig. 13]

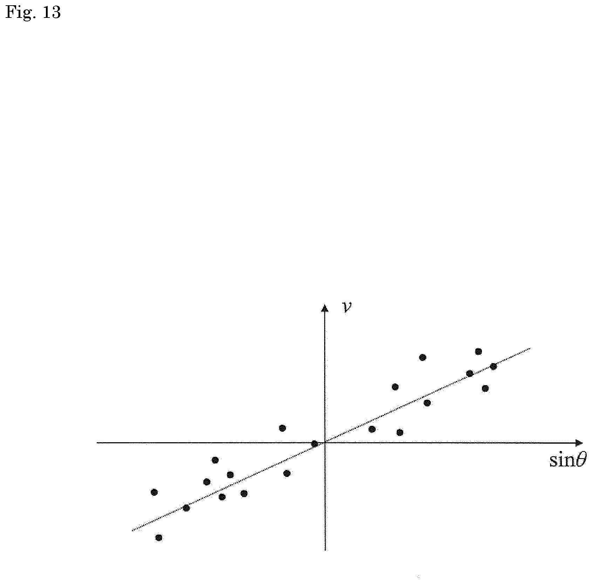

Fig. 13 is a diagram illustrating an example of a method for setting a position characteristic correction parameter.
[Fig. 14]

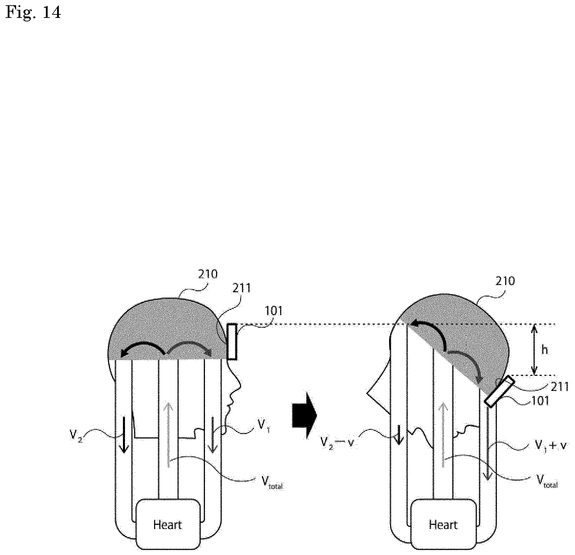

Fig. 14 is a model diagram illustrating changes in blood flow velocity caused by changes in the position of the head.
[Fig. 15]

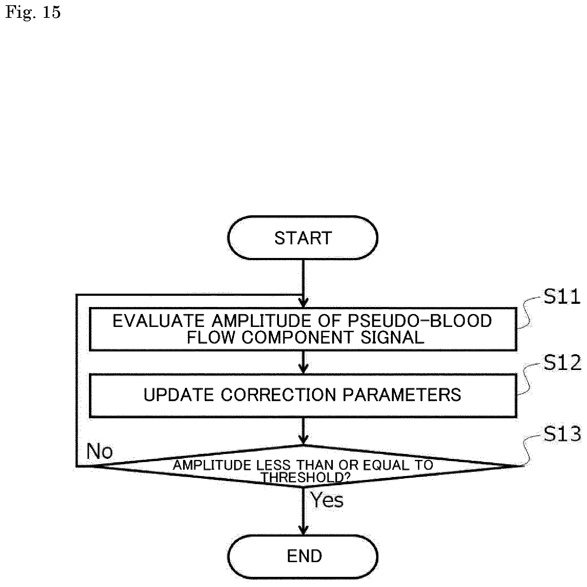

Fig. 15 is a flowchart illustrating an example of processing by a correction parameter updating unit 110 according to one embodiment of the present technique.
[Fig. 16]

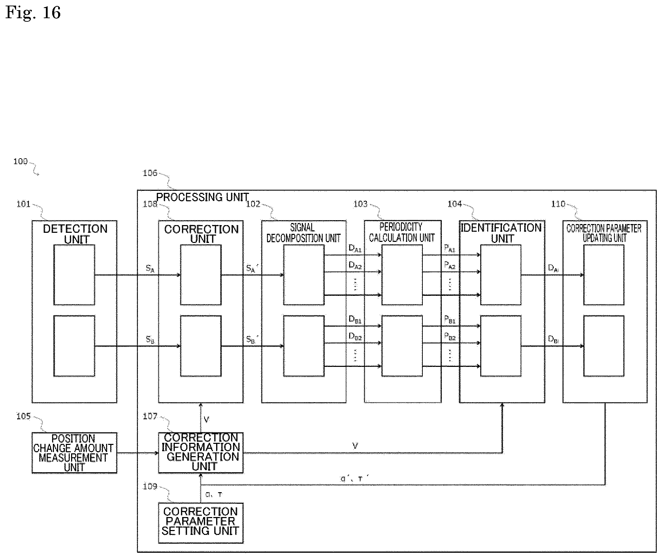

Fig. 16 is a block diagram illustrating the configuration of a biological information obtainment device 100 according to a fourth embodiment of the present technique.
[Fig. 17]

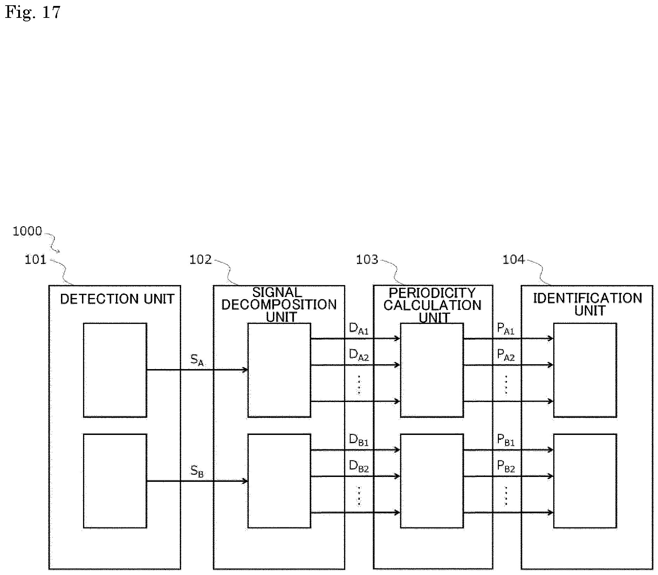

Fig. 17 is a block diagram illustrating the configuration of a biological information obtainment system 1000 according to a fifth embodiment of the present technique.
[Fig. 18]

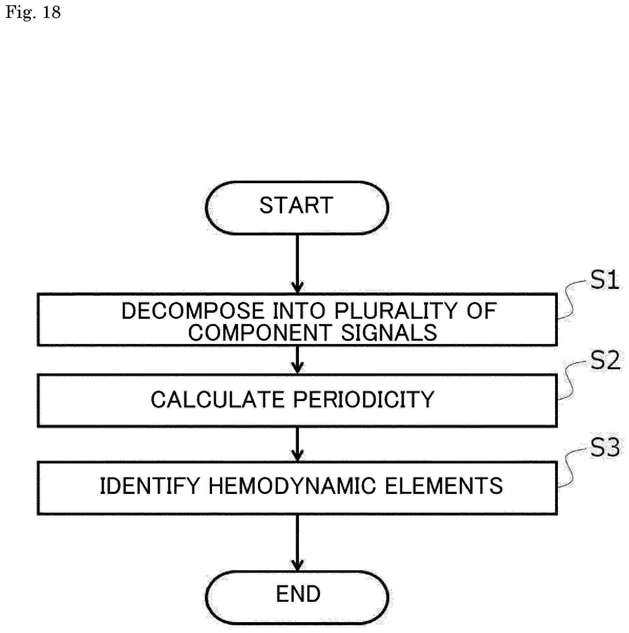

Fig. 18 is a flowchart illustrating an example of a sequence in a biological information obtainment method according to one embodiment of the present technique.
[Fig. 19]

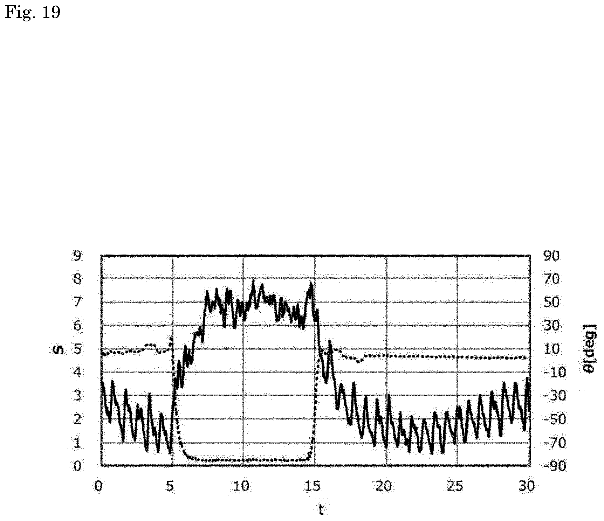

Fig. 19 is a graph showing a relationship between blood flow velocity and head tilt.
[Fig. 20]

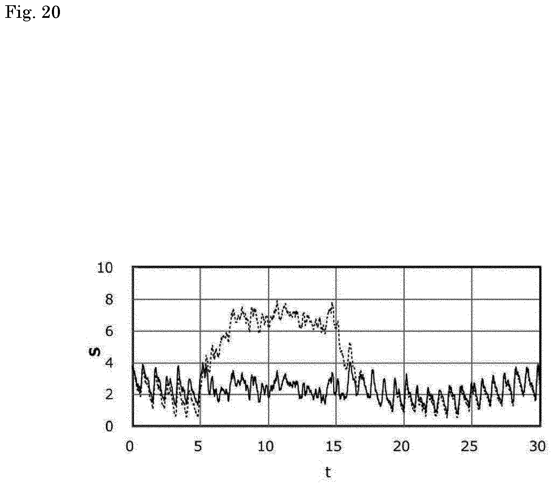

Fig. 20 is a graph showing blood flow velocity before and after correction.

[Description of Embodiments]

Preferred embodiments for carrying out the present technique will be described hereinafter. The following embodiments describe examples of representative embodiments of the present technique, and the scope of the present technique should not be narrowly interpreted on the basis thereof. Furthermore, the drawings are schematic diagrams, and are not necessarily exact illustrations.

In the drawings, unless otherwise specified, "up" means the upper direction or the upper side in the drawing, "down" means the lower direction or the lower side in the drawing, "left" means the left direction or the left side in the drawing, and "right" means the right direction or the right side in the drawing. Also, the same reference signs will be given to the same or equivalent elements or members in the drawings, and redundant descriptions thereof will not be given.

The present technique will be described in the following order.

1. First Embodiment of Present Technique (Example 1 of Biological Information Obtainment Device)
2. Second Embodiment of Present Technique (Example 2 of Biological Information Obtainment Device)
3. Third Embodiment of Present Technique (Example 3 of Biological Information Obtainment Device)
4. Fourth Embodiment of Present Technique (Example 4 of Biological Information Obtainment Device)
5. Fifth Embodiment of Present Technique (Biological Information Obtainment System)
6. Sixth Embodiment of Present Technique (Biological Information Obtainment Method)
7. Working Example

[1. First Embodiment of Present Technique (Example 1 of Biological Information Obtainment Device)]

[(1) Description of First Embodiment]

It is known that analyzing hemodynamic signals detected from organisms such as humans and animals makes it possible to estimate the health state and/or the psychological state of the human, for example.

However, hemodynamic signals tend to be a composite of multiple elements pertaining to hemodynamics. Hemodynamic signals tend to be composites of, for example, heartbeat component signals related to heartbeat fluctuations, vasomotion component signals related to mechanical interactions between blood flow and blood vessel walls, and/or other component signals. Accordingly, there is a problem in that the estimation accuracy drops when estimating the health state and/or psychological state of a human based on hemodynamic signals.

A biological information obtainment device according to one embodiment of the present technique is capable of accurately identifying hemodynamic elements present in hemodynamic signals detected from an organism. This will be described with reference to Fig. 1. Fig. 1 is a graph showing an example of a hemodynamic signal obtained by a biological information obtainment device 100 according to one embodiment of the present technique. As illustrated in Fig. 1, the biological information obtainment device according to one embodiment of the present technique can decompose a hemodynamic signal SA detected from an organism into a plurality of component signals DA1 to DA3, and can accurately identify respective elements of the plurality of component signals DA1 to DA3. To be more specific, the biological information obtainment device can identify the component signal DA1 as a heartbeat component signal. Alternatively, the biological information obtainment device can identify the component signal DA2 as a vasomotion component signal.

In other words, the hemodynamic elements identified by the biological information obtainment device according to one embodiment of the present technique can include a heartbeat component and/or a vasomotion component. Alternatively, the elements may include a pseudo-blood flow component, as will be described later in a third embodiment. If the component signal can be identified as a heartbeat component signal, information such as, for example, the blood oxygen saturation, the heart rate, and the like of a human can be obtained with high accuracy. If the component signal can be identified as a vasomotion component signal, information such as, for example, cutaneous blood flow rhythm, which is said to reflect the mental/physical state of a human, can be obtained with high accuracy. This cutaneous blood flow rhythm can be used, for example, to evaluate autonomous nerve system activity, confirm the effects of autonomic blockade surgery, and the like.

Note that these effects are the same in other embodiments described later. As such, these descriptions will not be repeated when describing other embodiments.

[(2) Configuration of First Embodiment]

The configuration of a biological information obtainment device 100 according to a first embodiment of the present technique will be described with reference to Fig. 2. Fig. 2 is a block diagram illustrating the configuration of the biological information obtainment device 100 according to the first embodiment of the present technique. As illustrated in Fig. 2, the biological information obtainment device 100 according to the first embodiment of the present technique can include, for example, a signal decomposition unit 102, a periodicity calculation unit 103, an identification unit 104, and the like.

The signal decomposition unit 102 decomposes a hemodynamic signal $S_A$ detected from an organism into a plurality of component signals ($D_{A1}$, $D_{A2}$, and the like). The periodicity calculation unit 103 calculates a periodicity of each of the plurality of component signals obtained from the decomposing by the signal decomposition unit 102, and obtains evaluation values ($P_{A1}$, $P_{A2}$, and the like). The identification unit 104 identifies hemodynamic elements based on the periodicity calculated by the periodicity calculation unit. The identification unit 104 can, as a result of the periodicity calculation unit 103 calculating the periodicity, accurately identify the hemodynamic elements. Each of these constituent elements will be described in detail hereinafter.

[(2-1) Signal Decomposition Unit]

The signal decomposition unit 102 decomposes a hemodynamic signal $S_A$ detected from an organism into a plurality of component signals ($D_{A1}$, $D_{A2}$, and the like). Although the method for the decomposition is not particularly limited, for example, Empirical Mode Decomposition (EMD), frequency decomposition, Short Time Fourier Transform (STFT), Wavelet Transform (WT), or the like can be used. Alternatively, Blind Source Separation (BSS) such as Principal Component Analysis (PCA), Independent Component Analysis (ICA), or the like may be used.

The signal decomposition unit 102 according to the present embodiment uses Empirical Mode Decomposition (EMD) as an example of the method for decomposition. EMD is a method used in fields such as signal processing, and is a method which converts time-series signals into non-stationary and nonlinear signals in the time-frequency domain. The signal decomposition unit 102 decomposes the hemodynamic signal into the sum and residual of a plurality of

Intrinsic Mode Functions (IMF) using the EMD algorithm. Each of the plurality of IMFs is a component signal and tends to be output in order of signals having a higher number of extreme values. In this manner, the signal decomposition unit 102 can effectively decompose a hemodynamic signal into component signal such as, for example, a heartbeat component signal and a vasomotion component signal using the EMD algorithm.

[(2-2) Periodicity Calculation Unit]

The periodicity calculation unit 103 calculates a periodicity of each of the plurality of component signals obtained from the decomposing by the signal decomposition unit 102. Features of the component signals are obtained as a result. The method for calculating the periodicity is not particularly limited, and for example, autocorrelation or the like may be calculated as the periodicity. In other words, the periodicity includes autocorrelation. An autocorrelation $R(\tau)$ is calculated according to the following Formula (1) using a value $v(i)$ of the component signal at time i, a delay time $\tau$, and a number N of sampling data contained in the component signal. [Math. 1]

$$R(\tau) = \frac{1}{N} \sum_{i=1}^{N} v(i) \cdot v(i + \tau) \quad \cdots \quad (1)$$

An example of the autocorrelation calculated using the component signal will be described with reference to Fig. 3. Fig. 3 is a graph showing examples of component signals and autocorrelation obtained by the biological information obtainment device 100 according to one embodiment of the present technique. Fig. 3A is a graph showing an example of a heartbeat component signal $D_{A1}$ and an autocorrelation $W_1$ calculated according to the foregoing Formula (1) using the heartbeat component signal $D_{A1}$. Fig. 3B is a graph showing an example of a vasomotion component signal $D_{A2}$ and an autocorrelation $W_2$ calculated according to the foregoing Formula (1) using the vasomotion component signal $D_{A1}$.

As illustrated in Fig. 3, the periodicity (autocorrelation) differs depending on the component signal. For example, for the heartbeat component signal $D_{A1}$, the value of the autocorrelation $W_1$ tends to be stable and high. This is because the heartbeat component signal changes continuously with the beating of the heart. On the other hand, for the vasomotion component signal $D_{A2}$, the value of the autocorrelation $W_2$ tends to fluctuate in an unstable manner. This is because the vasomotion component signal is affected by multiple physiological factors. These multiple physiological factors include, for example, cardiac, respiratory, myogenic, neurogenic, non-independent endothelial, and independent endothelial factors.

In this manner, features of the component signals can be obtained with high accuracy by the periodicity calculation unit 103 calculating the periodicity. As a result, the identification unit 104 can accurately identify hemodynamic elements. In particular, the identification unit 104 can identify whether a component signal is a heartbeat component signal, or whether a component signal is a vasomotion component.

Note that the autocorrelation $R(\tau)$ may be calculated according to the following Formula (2) using the value $v(i)$ of the component signal at time i, the delay time $\tau$, and the number N of sampling data contained in the component signal. [Math. 2]

$$R(\tau) = \frac{1}{N} \sum_{i=1}^{N} \left( v(i) - v(i + \tau) \right)^2 \quad \cdots \quad (2)$$

Note also that the periodicity calculation unit 103 may calculate the periodicity for a signal obtained by adding together the values of a plurality of component signals.

[(2-3) Identification Unit]

The identification unit 104 identifies hemodynamic elements based on the periodicity calculated by the periodicity calculation unit 103. For example, when the value of the autocorrelation calculated by the periodicity calculation unit 103 tends to be stable and high, the identification unit 104 can identify the component signal as a heartbeat component signal. Alternatively, when the value of the autocorrelation calculated by the periodicity calculation unit 103 tends to fluctuate in an unstable manner, the identification unit 104 can identify the component signal as a vasomotion component signal. The identification unit 104 can label the component signals with the identification results.

Note that a predetermined threshold may be used in this identification. For example, when the value of the autocorrelation calculated by the periodicity calculation unit 103 tends to be higher than the threshold, the identification unit 104 can identify the component signal as a heartbeat component signal.

As described above, the component signals (IMF) obtained from the decomposing performed by the signal decomposition unit 102 using the EMD algorithm tend to be output in order from signals having a higher number of extreme values. Accordingly, the identification unit 104 may identify hemodynamic elements based on the order in which the component signals are output. For example, the identification unit 104 may identify the first component signal output from the hemodynamic signal as a heartbeat component signal.

Alternatively, for example, when the signal decomposition unit 102 decomposes the hemodynamic signal into component signals using BSS, the identification unit 104 may identify the hemodynamic elements based on the contribution ratio of PCA, ICA, or the like.

A learning model trained by machine learning may be used for the identification unit 104. This learning model may be a supervised learning model that uses supervisory data including component signals, which are the input of the identification unit 104, and identification result data, which is the output of the identification unit 104. This learning model can be realized by using a neural network, a decision tree model, or the like, for example.

Although not illustrated in the drawings, the biological information obtainment device 100 may include a storage unit that stores data, a control unit that controls the constituent elements, and the like. A publicly-known storage technique (described later) can be used for the storage unit. For example, a central processing unit (CPU) (described later) or the like can be used for the control unit.

[(2-4) Hardware Configuration]

The hardware configuration of the biological information obtainment device 100 will be described with reference to Fig. 4. Fig. 4 is a block diagram illustrating the hardware configuration of the biological information obtainment device 100 according to one embodiment of the present technique. As illustrated in Fig. 4, the biological information obtainment device 100 can include, as constituent elements, a CPU 301, storage 302, a random access memory (RAM) 303, an operation unit 304, a display unit 305, and the like, for example. Elements which are publicly known in this technical field may be employed as these constituent elements. The respective constituent elements are connected by a bus serving as a data transmission line, for example.

The CPU 301 is realized by, for example, a microcomputer, and controls each constituent element of the biological information obtainment device 100. The CPU 301 can function as the signal decomposition unit 102, the periodicity calculation unit 103, the identification unit 104, or the like, for example. The signal decomposition unit 102 and the like can be realized by programs, for example. These programs can function by being loaded into the CPU 301.

The storage 302 stores programs used by the CPU 301, control data such as computation parameters, and the like. For example, a hard disk drive (HDD), a solid state drive (SSD), or another type of non-volatile memory may be employed as the storage 302.

The RAM 303 temporarily stores programs and the like executed by the CPU 301, for example.

The operation unit 304 accepts operations from the user. For example, a keyboard, a mouse, a trackball, a tablet, a touchpad, a stick-type pointing device, a touch panel, a joystick, or the like may be employed as the operation unit 304.

The display unit 305 provides the user with the status of processing performed by the CPU 301 and the like as images. For example, a display or the like may be employed as the display unit 305.

Although not illustrated in the drawings, the biological information obtainment device 100 may include, for example, a connection unit for connecting to an external computer device, a communication unit for communicating with an external computer device, and the like. The external computer device may be a detection unit, for example (described later). The communication unit has a function for data communication with the external computer device over an information communication network 400 using a communication technique such as, for example, Wi-Fi, Bluetooth (registered trademark), Long Term Evolution (LTE), or the like.

The biological information obtainment device 100 may be, for example, a PC, a server, a smartphone terminal, a tablet terminal, a mobile phone terminal, a Personal Digital Assistant (PDA), a Personal Computer (PC), a portable music player, a portable game console, or a wearable terminal (Head Mounted Display (HMD), a glasses-type HMD, a watch-type terminal, a band-type terminal, or the like).

The programs that realize the signal decomposition unit 102 and the like may be stored in a computer device or a computer system aside from the biological information obtainment device 100. In this case, the biological information obtainment device 100 can use a cloud service that provides the functions of the programs. Software as a Service (SaaS), Infrastructure as a Service (IaaS), Platform as a Service (PaaS), and the like can be given as examples of such cloud services.

Furthermore, the program can be stored and supplied to the computer using various types of non-transitory computer-readable media. Non-transitory computer-readable media include various types of tangible storage media. Examples of non-transitory computer-readable media include magnetic recording media (e.g., floppy disks, magnetic tape, and hard disk drives), magneto-optical recording media (e.g., magneto-optical discs), Compact Disc Read Only Memory (CD-ROM), CD-R, CD-R/W, and semiconductor memory (for example, Mask ROM, Programmable ROM (PROM), Erasable PROM (EPROM), Flash ROM, and Random Access Memory (RAM)). The program may also be supplied to the computer by various types of transitory computer readable media. Examples of transitory computer-readable media include electrical signals, optical signals, and electromagnetic waves. Transitory computer-readable media can deliver the program to the computer via wired communication channels, such as electrical wires and optical fibers, or wireless communication channels.

Note that the technology used in the present embodiment can also be used in other embodiments described later. The same applies to other embodiments.

[(2-5) Detection Unit]

The biological information obtainment device 100 according to one embodiment of the present technique can further include a detection unit that detects a hemodynamic signal from an organism. The detection unit may be included in the biological information obtainment device 100, or may be included in a device separate from the biological information obtainment device 100.

The method for detecting hemodynamic signals used by the detection unit may be a method known in this technical field. The detection method may be, for example, a Laser Doppler Flowmetry (LDF) method, a method using ultrasound waves, a method using electromagnetic induction, a photoplethysmography (PPG) method, or the like, for example. Alternatively, a remote sensor or the like such as a camera capable of obtaining hemodynamics detected through such methods may be employed as the detection unit.

A more specific example of the detection unit 101 detecting a hemodynamic signal using the LDF method will be described with reference to Fig. 5. Fig. 5 is a block diagram illustrating the configuration of the detection unit 101 according to one embodiment of the present technique. As illustrated in Fig. 5, the detection unit 101 can include, for example, a light emitting unit 111 that irradiates the skin surface of an organism with light, and a light receiving unit 112 that receives light resulting from irradiating the skin surface of the organism with the light. When the LDF method is used, the light may be coherent light, for example. In particular, the coherent light may be laser light, for example. Note that the light need not be coherent light when using the PPG method or the like, for example.

Although not illustrated in the drawings, the detection unit 101 may further include a blood flow velocity calculation unit that calculates a blood flow velocity based on the wavelength of scattered light received by the light receiving unit 112. Of scattered light produced by the emission of the coherent light, the frequency of scattered light scattered by biological tissue at rest is the same as the frequency of the emitted coherent light. On the other hand, the frequency of scattered light scattered by a scattering medium moving in the blood vessels of the skin of an organism (mainly red blood cells) undergoes a slight Doppler shift. Interference light produced by interference between these two instances of scattered light is received by the light receiving unit. The blood flow velocity calculation unit can calculate the blood flow velocity by performing frequency analysis processing on optical beats of the interference light. At this time, the hemodynamic signal detected by the detection unit 101 includes the blood flow velocity.

The LDF method is a method which is non-invasive and which enables continuous measurement. The detection unit 101 employing the LDF method can be small in size. Accordingly, the detection unit 101 can be attached to, for example, a fingertip, a wrist, an earlobe, the forehead, or the like of a human, an animal, or the like. The detection unit 101 can measure the blood flow velocity at each attachment position for an extended period of time.

[2. Second Embodiment of Present Technique (Example 2 of Biological Information Obtainment Device)]

[(1) Description of Second Embodiment]

The detection unit 101 may include at least two light receiving units. This will be described with reference to Fig. 6. Fig. 6 is a schematic diagram illustrating the detection unit 101 according to one embodiment of the present technique. Fig. 6A is a schematic plan view of the detection unit 101. Fig. 6B is a schematic side view of the detection unit 101.

Each of a plurality of light receiving units 112A to 112G receives scattered light scattered by a scattering medium moving through different blood vessels. Accordingly, the detection unit 101 can calculate the velocity of the blood flow through each of the different blood vessels. Note that the number of light receiving units is not particularly limited. There may be two light receiving units, or seven, as illustrated in Fig. 6A.

By providing the detection unit 101 with a plurality of light receiving units, the biological information obtainment device 100 can obtain a correlation between hemodynamic signals detected from different blood vessels. In particular, the biological information obtainment device 100 can obtain a correlation between heartbeat component signals and/or vasomotion component signals included in the hemodynamic signals.

The respective characteristics of the heartbeat component signal and the vasomotion component signal will be described with reference to Fig. 7. Fig. 7 is a graph showing examples of component signals obtained by the biological information obtainment device 100 according to one embodiment of the present technique. Fig. 7A is a graph showing an example of the heartbeat component signals $D_{A1}$ and $D_{B1}$. Fig. 7B is a graph showing an example of the vasomotion component signals $D_{A2}$ and $D_{B2}$.

In Fig. 7, the hemodynamic signal obtained through the light receiving unit 112A (see Fig. 6), for example, is taken as a hemodynamic signal pertaining to a channel A. Likewise, the hemodynamic signal obtained through the light receiving unit 112B (see Fig. 6) is taken as a hemodynamic signal pertaining to a channel B. By analyzing the correlation between the hemodynamic signal pertaining to channel A and the hemodynamic signal pertaining to channel B, the identification unit 104 can identify the hemodynamic elements.

[(2) Calculation of Periodicity]

For example, PTL 1 describes a technique that reduces motion artifacts by using a combination of sensor data pertaining to two channels.

However, this technique has a problem in that a heartbeat component and/or a vasomotion component cannot be accurately identified simply by analyzing a correlation between signals. This will be described with reference to Fig. 7. As illustrated in Fig. 7A, there is an overall high correlation between the heartbeat component signal $D_{A1}$ pertaining to channel A and the heartbeat component signal $D_{B1}$ pertaining to channel B. On the other hand, as illustrated in Fig. 7B, the correlation between the vasomotion component signal $D_{A2}$ pertaining to channel A and the vasomotion component signal $D_{B2}$ pertaining to channel B has a region H in which the correlation is relatively high and a region L in which the correlation is relatively low. This is because, as described above, the vasomotion component signal is affected by multiple physiological factors.

To solve this problem, first, the signal decomposition unit 102 decomposes a first hemodynamic signal and a second hemodynamic signal, which have been detected from at least two locations of an organism, into a plurality of component signals each. The first hemodynamic signal may be the hemodynamic signal $S_A$ pertaining to channel A. The second hemodynamic signal may be the hemodynamic signal $S_B$ pertaining to channel B. In other words, the signal decomposition unit 102 decomposes the hemodynamic signal $S_A$ pertaining to channel A into a plurality of component signals ($D_{A1}$, $D_{A2}$, and the like). Additionally, the signal decomposition unit 102 decomposes the hemodynamic signal $S_B$ pertaining to channel B into a plurality of component signals ($D_{B1}$, $D_{B2}$, and the like).

Next, the periodicity calculation unit 103 calculates a periodicity of each of the plurality of component signals. This will be described with reference to Fig. 8. Fig. 8 is a graph showing examples of component signals obtained by the biological information obtainment device 100 according to one embodiment of the present technique. Fig. 8A is a graph showing an example of the heartbeat component signals ($D_{A1}$ and $D_{B1}$) and autocorrelations ($W_{A1}$ and $W_{B1}$). Fig. 8B is a graph showing an example of the vasomotion component signals ($D_{A2}$ and $D_{B2}$) and autocorrelations ($W_{A2}$ and $W_{B2}$).

The autocorrelation $W_{A1}$ of the heartbeat component signal $D_{A1}$ pertaining to channel A and the autocorrelation $W_{B1}$ of the heartbeat component signal $D_{B1}$ pertaining to channel B both tend to have high and stable values. This is because heartbeat component signals change continuously with the beating of the heart, and have a characteristic of changing uniformly regardless of the blood vessel.

On the other hand, for the autocorrelation $W_{A2}$ of the vasomotion component signal $D_{A2}$ pertaining to channel A and the autocorrelation $W_{B2}$ of the vasomotion component signal $D_{B2}$ pertaining to channel B, the tendency of the values to fluctuate differs depending on the channel. This is because, as described above, the vasomotion component signal is affected by multiple physiological factors. This is particularly because vasomotion component signals are affected by the muscles near the corresponding vessels.

[(3) Identifying Hemodynamic Elements]

The identification unit 104 uses these characteristics for identification. According to the invention, the identification unit 104 identifies hemodynamic elements based on the correlation between the periodicity of the component signals included in the first hemodynamic signal pertaining to channel A and the periodicity of the component signals included in the second hemodynamic signal pertaining to channel B. For example, the identification unit 104 can identify a component signal as a heartbeat component signal when the correlation is high, and can identify a component signal as a vasomotion component signal when the correlation is low.

As described above, the component signals (Intrinsic Mode Function (IMF)) obtained from the decomposing performed by the signal decomposition unit 102 using the EMD algorithm tend to be output in order from signals having a higher number of extreme values. Accordingly, the identification unit 104 can identify hemodynamic elements based on the correlation between periodicities of respective component signals in the order in which the component signals are output. Specifically, the identification unit 104 identifies hemodynamic elements based on the correlation between the periodicity of the first component signal $D_{A1}$ output from the first hemodynamic signal $S_A$ and the periodicity of the first component signal $D_{B1}$ output from the second hemodynamic signal $S_B$. Next, the identification unit 104 identifies hemodynamic elements based on the correlation between the periodicity of the second component signal $D_{A2}$ output from the first hemodynamic signal $S_A$ and the periodicity of the second component signal $D_{B2}$ output from the second hemodynamic signal $S_B$. In this manner, the identification unit 104 can identify the component signals in the order in which they are output.

According to the invention, an evaluation value E of the correlation is calculated through the following Formula (3) using an evaluation value $P_{A1}$ of the periodicity of the component signal $D_{A1}$ included in the first hemodynamic signal $S_A$, an evaluation value $P_{B1}$ of the periodicity of the component signal $D_{B1}$ included in the second hemodynamic signal $S_B$, and the number N of sampling data contained in the component signals. Note that the evaluation value of the periodicity may be an autocorrelation value, for example. [Math. 3]

$$E = \sum_{i=1}^{N} \left( P_{A1}(i) - P_{B1}(i) \right) \quad \cdots \quad (3)$$

As described above, the identification unit 104 can identify a component signal as a heartbeat component signal when the correlation is high, and can identify a component signal as a vasomotion component signal when the correlation is low. Accordingly, for example, the identification unit 104 can identify a component signal for which the correlation evaluation value E is the highest as a heartbeat component, and can identify a component signal for which the correlation evaluation value E is the lowest as a vasomotion component.

Alternatively, a predetermined threshold may be set. Accordingly, for example, the identification unit 104 may identify a component signal for which the correlation evaluation value E is higher than the threshold as a heartbeat component, and may identify a component signal for which the correlation evaluation value E is lower than the threshold as a vasomotion component.

Alternatively, the identification unit 104 may calculate the correlation evaluation value E using a combination of the component signals in a plurality or all of the hemodynamic signals.

[3. Third Embodiment of Present Technique (Example 3 of Biological Information Obtainment Device)]

[(1) Description of Third Embodiment]

As described above, the detection unit 101 employing the LDF method or the like can be small in size. Accordingly, the detection unit 101 can be attached to, for example, a fingertip, a wrist, an earlobe, the forehead, or the like of a human, an animal, or the like. The detection unit 101 can measure the blood flow velocity included in the hemodynamic signal at each attachment position for an extended period of time.

However, this blood flow velocity fluctuates not only with the health state and/or psychological state of the human or animal, but also with the posture of the human or animal. For example, a phenomenon has been observed in which when the detection unit 101 is attached to the forehead, the blood flow velocity in the forehead increases when the head faces downward. In this case, it is difficult to determine, based on changes in the blood flow velocity alone, whether a fluctuation in the blood flow velocity is caused by the health state and/or psychological state, or has arisen simulatively due to a change in posture. In other words, a pseudo-blood flow component signal arising simulatively due to a change in posture is noise, and makes it difficult to accurately obtain a blood flow velocity caused by the health state and/or psychological state.

PTL 1 describes a technique that reduces motion artifacts by using body motion reference data. However, when measuring blood flow velocity, a pseudo-blood flow component signal such as that described above arises, which makes it difficult to use body motion reference data.

Accordingly, in the present technique, blood flow velocity which fluctuates depending on the health state and/or psychological state is accurately obtained by identifying and removing a pseudo-blood flow component signal included in the hemodynamic signal.

The biological information obtainment device 100 according to one embodiment of the present technique generates correction information based on a change amount in the position of a detection unit placed on the head, and corrects the blood flow velocity measured by the detection unit using the correction information. This makes it possible to remove, from the measured blood flow velocity of the head, fluctuations in the blood flow velocity caused by changes in the position of the head, i.e., fluctuations in the blood flow velocity caused by changes in the posture of a human, an animal, or the like. As such, the biological information obtainment device 100 according to one embodiment of the present technique can obtain a hemodynamic signal which makes it possible to more accurately ascertain the health state and/or psychological state of a human, animal, or the like.

[(2) Configuration of Third Embodiment]

The configuration of a biological information obtainment device 100 according to the third embodiment of the present technique will be described with reference to Fig. 9. Fig. 9 is a block diagram illustrating the configuration of the biological information obtainment device 100 according to the third embodiment of the present technique.

As illustrated in Fig. 9, the biological information obtainment device 100 according to the third embodiment of the present technique can include, for example, the detection unit 101, a position change amount measurement unit 105, a processing unit 106, and the like. The processing unit 106 can include, for example, a correction unit 108, the signal decomposition unit 102, the identification unit 104, a correction parameter updating unit 110, a correction information generation unit 107, a correction parameter setting unit 109, and the like. Note that the detection unit 101 and the like, for example, may be provided in a device separate from the biological information obtainment device 100.

The detection unit 101 may be attached to the head, for example. The detection unit 101 may be attached to any position of the head, e.g., the front of the head, the side of the head, or the rear of the head. The detection unit 101 is configured to be capable of measuring the blood flow velocity in the head.

The position change amount measurement unit 105 measures an amount of change in the position of the detection unit 101. In the present technique, the amount of change may be an amount of change in the tilt of the detection unit 101 or an amount of change in the height of the detection unit 101.

The position change amount measurement unit 105 may include, for example, an accelerometer, a gyrosensor, or an atmospheric pressure sensor. These sensors which can be used in the present technique may be sensors which are known in this technical field. For example, one of these sensors may be provided in the biological information obtainment device 100 so as to change position in the same manner as the detection unit 101. For example, the detection unit 101 and the position change amount measurement unit 105 having a fixed positional relationship makes it possible to treat an amount of change in the position measured by the position change amount measurement unit 105 as an amount of change in the position of the detection unit 101.

Alternatively, the position change amount measurement unit 105 may be a combination of a marker indicating the position of the detection unit 101 and a marker recognition device that recognizes the marker. The marker may be attached to the detection unit 101, or may be attached to the biological information obtainment device 100 so as to change position in the same manner as the detection unit 101. The marker recognition device may include an image sensor. The position change amount measurement unit 105 can measure an amount of change in the position of the marker by processing still images or moving images obtained by the image sensor. The position change amount measurement unit 105 can treat the amount of change in the position of the marker as the amount of change in the position of the detection unit 101.

The processing unit 106 can include a processor such as a CPU, for example, as well as memory such as RAM and/or ROM, for example. Programs for causing a device to execute biological information obtainment processing according to the present technique, a position characteristic correction parameter and a transient characteristic correction parameter (described later), a program for setting or updating these parameters, and the like can be stored in the memory. The processor can realize the functions of the processing unit 106 by loading this program and the like.

The correction information generation unit 107 generates blood flow velocity correction information based on the amount of change in the position of the detection unit 101 measured by the position change amount measurement unit 105. This correction information is generated based on the amount of change in the position of the detection unit 101, and is therefore suitable for canceling out changes (increases or decreases) in the blood flow velocity caused by changes in the position of the head.

In other words, according to one embodiment of the present technique, the correction information generation unit 107 generates correction information for canceling out an increase/decrease in blood flow velocity caused by a change in the position of the head. For example, the correction information generation unit 107 generates correction information for canceling out an increase in the blood flow velocity caused by the position of the head dropping, or generates correction information for canceling out a decrease in the blood flow velocity caused by the position of the head rising. More particularly, the correction information generation unit 107 generates the correction information based on the amount of change in the position, as well as a position characteristic correction parameter set based on the correlation between the position of the detection unit and the amount of change in blood flow velocity and/or a transient characteristic correction parameter pertaining to transient characteristics of the blood flow velocity. Note that specific examples of the correction information generated will be described below in (2-1) and (2-2).

Using the correction information generated by the correction information generation unit 107, the correction unit 108 corrects the blood flow velocity included in the hemodynamic signal detected by the detection unit 101. For example, if the blood flow velocity has increased due to the position of the head dropping, the correction unit 108 uses the correction information generated by the correction information generation unit 107 to subtract the amount of the increase from the measured blood flow velocity. Alternatively, if the blood flow velocity has decreased due to the position of the head rising, the correction unit 108 uses the correction information generated by the correction information generation unit 107 to add the amount of the decrease to the measured blood flow velocity. In this manner, in the present technique, the correction unit 108 uses the correction information to cancel out an increase/decrease in the blood flow velocity caused by a change in the position of the head. For example, using the correction information, the correction unit 108 can cancel out an increase in the blood flow velocity caused by the position of the head dropping, or can cancel out a decrease in the blood flow velocity caused by the position of the head rising.

The correction parameter setting unit 109 sets the position characteristic correction parameter set based on the correlation between the position of the detection unit 101 and the amount of change in the blood flow velocity, and/or the transient characteristic correction parameter pertaining to transient characteristics of the blood flow velocity. Note that the setting of the position characteristic correction parameter and/or the transient characteristic correction parameter will be described in detail in (2-1) or (2-2) below.

The correction parameter setting unit 109 can set and/or update the position characteristic correction parameter and/or the transient characteristic correction parameter before the correction by the correction unit 108, for example. The biological information obtainment device 100 may already have a predetermined position characteristic correction parameter and/or transient characteristic correction parameter, or may not already have a position characteristic correction parameter and/or a transient characteristic correction parameter.

If the biological information obtainment device 100 already has a predetermined position characteristic correction parameter and/or transient characteristic correction parameter, the position characteristic correction parameter and/or the transient characteristic correction parameter may be updated by the correction parameter setting unit 109 before the correction of the blood flow velocity according to the present technique is performed. For example, the position characteristic correction parameter and/or the transient characteristic correction parameter set when the biological information obtainment device 100 is shipped may be used as-is, or the position characteristic correction parameter and/or transient characteristic correction parameter set the first time a given user uses the biological information obtainment device 100 may be used as-is the next time the device is used (the second and subsequent times the biological information obtainment device or the detection unit 101 is worn).

If the biological information obtainment device 100 does not already have a position characteristic correction parameter and/or a transient characteristic correction parameter, the position characteristic correction parameter and/or the transient characteristic correction parameter may be set by the correction parameter setting unit 109 before the correction of the blood flow velocity according to the present technique is performed.

In this manner, by setting or updating the position characteristic correction parameter before the correction of the blood flow velocity according to the present technique is performed, the position characteristic correction parameter and/or transient characteristic correction parameter are set or updated according to the subject (and particularly, a human) whose blood flow is to be measured by the biological information obtainment device according to the present technique. More accurate correction information is generated as a result of the set or updated position characteristic correction parameter and/or transient characteristic correction parameter, and the blood flow velocity can be corrected more appropriately based on this more accurate correction information.

The position characteristic correction parameter and/or transient characteristic correction parameter may be updated at predetermined time intervals or every buffer unit while the biological information obtainment device 100 according to the present technique is being worn by the subject. To be more specific, the updates may be performed, for example, every three minutes to three hours; particularly, every five minutes to two hours; and more particularly, every 10 minutes to one hour. "Buffer unit" refers to one sample unit of blood flow velocity measured by the detection unit 101, and for example, a predetermined number of measurements of the blood flow velocity can be taken as one unit, or a predetermined time interval at which the blood flow velocity is measured can be taken as one unit. By updating the position characteristic correction parameter at predetermined time intervals or every buffer unit in this manner, the blood flow velocity can be corrected more appropriately by using a more appropriate position characteristic correction parameter and/or transient characteristic correction parameter when the biological information obtainment device according to the present technique obtains the blood flow velocity over an extended period of time, for example. Note that the setting of the position characteristic correction parameter and/or the transient characteristic correction parameter will be described in detail in (2-1) or (2-2) below.

The signal decomposition unit 102 and the identification unit 104 can use the techniques described in other embodiments. The signal decomposition unit 102 decomposes the hemodynamic signal corrected by the correction unit 108 into a plurality of component signals. The identification unit 104 identifies a pseudo-blood flow component signal from the plurality of component signals. The identification of the pseudo-blood flow component signal will be described in detail in (2-3) below.

The correction parameter updating unit 110 updates the position characteristic correction parameter and/or the transient characteristic correction parameter so as to reduce the pseudo-blood flow component signal, which acts as noise. The updating of the position characteristic correction parameter and/or the transient characteristic correction parameter will be described in detail in (2-4) below.

Although not illustrated in the drawings, the biological information obtainment device 100 may further include an output unit. The output unit can output the blood flow velocity obtained by the identification unit 104, a psychological state or a health state of the measurement subject determined based on the blood flow velocity, or video or audio based on the blood flow velocity. The output unit can include, for example, a printing device, an image display device, or an audio output device for outputting the blood flow velocity, the psychological state, the health state, the video, or the audio. The video or audio based on the blood flow velocity may, for example, be video or audio for notifying the measurement subject when the blood flow velocity exits a predetermined numerical range, or video or audio prompting the measurement subject to rest.

[(2-1) Correction Information Based on Amount of Change in Tilt]

According to one embodiment of the present technique, the correction information generation unit 107 generates the blood flow velocity correction information based on an amount of change in the tilt of the detection unit 101. In this embodiment, the tilt may be, for example, an angle projected onto a plane passing through the detection unit 101 and a centerline of the head. The generation of this correction information will be described in detail hereinafter.

The velocity of blood flow through the blood vessels in a given position of the head changes with changes in the position of the head. Changes in the blood flow velocity caused by changes in the position of the head (an increase in the blood flow velocity and a decrease in the blood flow velocity) will be described with reference to Fig. 10. Fig. 10 is a model diagram illustrating changes in the blood flow velocity.

As illustrated on the left side of Fig. 10, the detection unit 101 is attached to the front of the head (forehead) 211 of a human 210. The detection unit 101, which is positioned at the center of the front of the head 211, measures the blood flow velocity in the front of the head 211 through the LDF method.

On the left side of Fig. 10, the human 210 is facing forward, and the position of the detection unit 101 in the state is taken as a reference position. In the situation illustrated on the left side of Fig. 10, for example, blood flowing to the head from the heart is represented by $V_{total}$, the blood flow velocity in the skin at a given position of the front of the head measured by the detection unit 101 is represented by $V_1$, and the blood flow velocity in the skin at a given position of the rear side of the head is represented by $V_2$.

A state in which the human 210 faces down by an angle θ is illustrated on the right side of Fig. 10. The angle θ is an amount of change in the tilt of the detection unit 101. As illustrated in Fig. 10, the angle θ is an angle projected onto a plane P passing through the detection unit 101 and a centerline X of the head of the human 210. As illustrated on the right side of Fig. 10, when the head faces downward by the angle θ, the blood flow velocity at the given position of the front of the head measured by the detection unit 101 increases by a fluctuation amount v (i.e., becomes V1 + v), and the blood flow velocity in the vessels at a given position on the rear side of the head decreases by the fluctuation amount v (i.e., becomes V2 - v).

The fluctuation amount v will be explained with reference to Fig. 11. Fig. 11 is a model pertaining to resistance force and gravity acting on a red blood cell having a mass m. In Fig. 11, cv represents the resistance force acting on a red blood cell RBC, and this resistance force is caused by, for example, blood vessels or the like. In Fig. 11, mgsinθ represents the force produced by gravity acting on the red blood cell RBC. In this model, the fluctuation amount v is expressed by the equation of motion in the following Formula (4).

[Math. 4]

$$\mathrm{m}\frac{dv}{dt} = -cv + mg\sin\theta \quad \cdots \quad (4)$$

A variant on the foregoing Formula (4) is as follows.

[Math. 5]

$$dv = -\frac{c}{m}\left(v + \frac{mg}{c}\sin\theta\right)dt \quad \cdots \quad (5)$$

If c/m in Formula (5) is taken as τ and mg/c is taken as α, Formula (4) becomes the following Formula (6).

[Math. 6]

$$dv = -\tau\big(v + \alpha(\sin\theta)\big)dt \quad \cdots \quad (6)$$

By integrating both sides thereof, Formula (6) becomes the following Formula (7).
[Math. 7]

$$v = \alpha \times \sin\theta\, e^{-\tau/t} \quad \cdots \quad (7)$$

As indicated above, the fluctuation amount v is expressed by Formula (7).

In this manner, according to one embodiment of the present technique, the correction information for canceling out an increase in the blood flow velocity caused by the position of the head dropping may be the fluctuation amount v expressed by Formula (7). Additionally, the correction information for canceling out a decrease in the blood flow velocity caused by the position of the head rising may also be the fluctuation amount v expressed by Formula (7).

In other words, when the position of the detection unit 101 has dropped, subtracting the fluctuation amount v expressed by Formula (7) from the measured blood flow velocity eliminates the increase in the blood flow velocity caused by the change in the position of the head. When the position of the detection unit 101 has risen, adding the fluctuation amount v expressed by Formula (7) to the measured blood flow velocity compensates for the decrease in the blood flow velocity caused by the change in the position of the head.

$\alpha$ in Formula (7) is called the position characteristic correction parameter. The position characteristic correction parameter may be set or updated based on the relationship between the position of the detection unit and the amount of change in the blood flow velocity. The setting can be performed by the correction parameter setting unit 109. One example of a method for setting the position characteristic correction parameter will be described below.

The detection unit 101 is attached to the forehead of the human. With the detection unit 101 attached, the human changes the position of their head from the reference position to various positions. The various positions may include at least one, at least two, at least three, or four positions selected from, for example, a position when the human is facing downward, a position when a human is facing upward, a position when the head is tilted to the right, and a position when the head is tilted to the left. In addition to these positions, the various positions may include, for example, a position when the human is facing downward and the head is tilted to the right and/or the left, a position when a human is facing upward and the head is tilted to the right and/or the left, and the like.

The detection unit 101 measures the blood flow velocity at the forehead at each of the various positions, and the position change amount measurement unit 105 measures the amount of change in the position of the detection unit 101. The correction parameter setting unit 109 sets the position characteristic correction parameter based on the blood flow velocity measured at the various positions and the amount of change in the position. In other words, the correction parameter setting unit 109 sets the position characteristic correction parameter based on a relationship between the amount of change in the position when the position of the detection unit 101 is changed from the reference position to various different positions, and the blood flow velocity (and particularly, the amount of fluctuation in the blood flow velocity) measured at each of the various different positions.

The measurement of the blood flow velocity by the detection unit 101 may be performed once a predetermined period of time has passed following the change in the position of the head to each of the aforementioned various positions. The blood flow velocity changes with a time delay relative to changes in the position of the head. In other words, changes in the blood flow velocity caused by changes in the position of the head have transient characteristics. Accordingly, as described above, a more accurate blood flow velocity can be measured after the change in the position of the head by measuring the blood flow velocity once a predetermined period of time has passed following the change in the position of the head. The predetermined period of time can be, for example, from one to 60 seconds; particularly, from three to 40 seconds; and more particularly, from five to 30 seconds.

Alternatively, the position of the head may be changed so slowly that the transient characteristics can be ignored. The blood flow velocity may be measured after this slow change in the position of the head. If the position of the head is changed slowly in this manner, the movement of the head need not be stopped at a specific position. For example, the head may be slowly moved for one revolution, and the blood flow velocity may be measured sequentially during that one revolution.

$\theta$ in Formula (7) represents the amount of change in the tilt of the detection unit 101, and as illustrated in Fig. 12, may be an angle projected onto the plane P passing through the detection unit 101 and the centerline X of the head of the human 210. $\theta$ may be measured by the position change amount measurement unit 105, and may be measured by, for example, a three-axis accelerometer (not shown) included in the position change amount measurement unit 105. The position change amount measurement unit 105 may be disposed at the center of the front of the head 211 so that the position thereof changes in the same manner as the position of the detection unit 101. For example, by fixing the positional relationship between the position change amount measurement unit 105 and the detection unit 101 within the biological information obtainment device 100, the detection unit 101 and the position change amount measurement unit 105 undergo the same changes in position. This makes it possible to regard the amount of change in the position of the position change amount measurement unit 105 as the amount of change in the position of the detection unit 101.

θ in Formula (7) may be determined using the following Formula (8) based on output values of the three-axis accelerometer, for example.

[Math. 8]

$$
\theta = \frac{a_1 b_1 + a_2 b_2 + a_3 b_3}{\sqrt{a_1{}^2 + a_2{}^2 + a_3{}^2}\sqrt{b_1{}^2 + b_2{}^2 + b_3{}^2}} \qquad \cdots \quad (8)
$$

In Formula (8), a = [a1, a2, a3] represents the output values of the three-axis accelerometer when the human 210 is facing forward, i.e., the output values of the three-axis accelerometer at the reference position, as illustrated on the left side of Fig. 10. In Formula (8), b = [b1, b2, b3] represents the output values of the three-axis accelerometer when the head of the human 210 is facing downward at the angle θ, as illustrated on the right side of Fig. 10. In the present technique, the angle θ may be measured by a gyrosensor.

For example, the detection unit 101 measures the amount of fluctuation in the blood flow velocity, and the three-axis accelerometer measures the amount of change in the position, at each of the following: a position when the human is facing downward; a position when the human is facing upward; a position when the head is tilted to the right; a position when the head is tilted to the left; a position when the human is facing downward and the head is tilted to the right; a position when the human is facing downward and the head is tilted to the left; a position when the human is facing upward and the head is tilted to the right; and a position when the human is facing upward and the head is tilted to the left. These measurements may be taken multiple times at each position. The amount of fluctuation in the blood flow velocity and the amount of change in the position at each position are plotted on a vertical axis and a horizontal axis, respectively. The slope of a line obtained by applying the least-squares method to the plotted data may be employed as the position characteristic correction parameter α. For example, the plots and the line indicated in Fig. 13 are obtained by measuring the amount of fluctuation in the blood flow velocity and the amount of change in position at various positions. In Fig. 13, the vertical axis represents the fluctuation amount v of the blood flow, and the horizontal axis represents a position change amount sinθ.

τ in Formula (7) is called the transient characteristic correction parameter. The transient characteristic correction parameter τ is a parameter pertaining to the transient characteristics of the blood flow velocity. As described above, the blood flow velocity changes with a time delay relative to a change in the position of the head, i.e., changes in the blood flow velocity caused by changes in the position of the head have transient characteristics. The transient characteristic correction parameter τ is used to reflect these transient characteristics in the measurement value of the blood flow velocity.

The transient characteristic correction parameter τ may be calculated, for example, by fitting a blood flow velocity estimated value estimated using the position characteristic correction parameter α to transient blood flow velocity data measured over time as the position of the detection unit 101 changes from the reference position to another position. The fitting may be performed through linear fitting or non-linear fitting, for example. The correction parameter setting unit 109 may calculate the transient characteristic correction parameter as described above and set that parameter, for example.

[(2-2) Correction Information Based on Amount of Change in Height]

According to another embodiment of the present technique, the correction information generation unit 107 may generate the blood flow velocity correction information based on an amount of change in the height of the detection unit 101. A specific example of the generation of the correction information will be described hereinafter.

Changes in the blood flow velocity caused by changes in the position of the head (an increase in the blood flow velocity and a decrease in the blood flow velocity) will be described with reference to Fig. 14. Fig. 14 is a model diagram illustrating changes in the blood flow velocity. In Fig. 14, the change in the position of the detection unit 101 is indicated using a height h instead of the angle θ in Fig. 10. In other words, the left side of Fig. 14 illustrates a state where the human 210 is facing forward, i.e., the detection unit 101 is in the reference position. The right side of Fig. 14 illustrates a case where the position of the head 211 has dropped by h, and thus the position of the detection unit 101 has also dropped by h.

On the left side of Fig. 14, the human 210 is facing forward, and the position of the detection unit 101 in the state is taken as a reference position. In the situation illustrated on the left side of Fig. 14, for example, blood flowing to the head from the heart is represented by $V_{total}$, the blood flow velocity at a given position of the front of the head measured by the detection unit 101 is represented by $V_1$, and the blood flow velocity in the blood vessels at a given position of the rear side of the head is represented by $V_2$.

As illustrated on the right side of Fig. 14, the height of the front of the head 211 of the human 210 has dropped by h, and as a result, the position of the detection unit 101 has also dropped by the height h. When the position of the head drops by the height h, the blood flow velocity at the given position of the front of the head measured by the detection unit 101 increases by a fluctuation amount v (i.e., becomes V1 + v), and the blood flow velocity in the vessels at a given position on the rear side of the head decreases by the fluctuation amount v (i.e., becomes V2 - v).

The fluctuation amount v can be expressed by an equation of motion using mgh instead of mgsinθ in the foregoing Formula (4). Transforming this equation of motion through a transformation similar to that from the foregoing Formula (4) to Formula (7) results in the following Formula (9).

[Math. 9]

$$v = \alpha \times he^{-\tau/t} \quad \cdots \quad (9)$$

In this manner, according to one embodiment of the present technique, the correction information for canceling out an increase in the blood flow velocity caused by the position of the head dropping may be the fluctuation amount v expressed by Formula (9). Additionally, the correction information for canceling out a decrease in the blood flow velocity caused by the position of the head rising may also be the fluctuation amount v expressed by Formula (9).

In other words, when the position of the detection unit 101 has dropped, subtracting the fluctuation amount v expressed by Formula (9) from the measured blood flow velocity eliminates the increase in the blood flow velocity caused by the change in the position of the head. When the position of the detection unit 101 has risen, adding the fluctuation amount v expressed by Formula (9) to the measured blood flow velocity makes it possible to compensate for the decrease in the blood flow velocity caused by the change in the position of the head.

Like $\alpha$ in Formula (7), $\alpha$ in Formula (9) is called a position characteristic correction parameter. The position characteristic correction parameter may be set based on the relationship between the position of the detection unit and the amount of change in the blood flow velocity. The correction parameter setting unit 109 can set and/or update the position characteristic correction parameter based on the blood flow velocity measured at various specific positions and the amount of change in the position. In other words, the correction parameter setting unit 109 can set and/or update the position characteristic correction parameter based on a relationship between the amount of change in the position when the position of the detection unit is changed from the reference position to various different positions, and the blood flow velocity (and particularly, the amount of fluctuation in the blood flow velocity) measured at each of the various different positions. One example of a method for setting the position characteristic correction parameter will be described below.

The detection unit 101 is attached to the forehead of the human. With the detection unit 101 attached, the human changes the position of their head from the reference position to various positions.

The various positions may include at least one, at least two, at least three, or four positions selected from, for example, a position when the human is facing downward, a position when a human is facing upward, a position when the head is tilted to the right, and a position when the head is tilted to the left. In addition to these positions, the various positions may include, for example, a position when the human is facing downward and the head is tilted to the right and/or the left, a position when a human is facing upward and the head is tilted to the right and/or the left, and the like.

The detection unit 101 measures the blood flow velocity at the forehead at each of the various positions, and the position change amount measurement unit 105 measures the amount of change in the position of the detection unit 101.

The measurement of the blood flow velocity by the detection unit 101 may be performed once a predetermined period of time has passed following the change in the position of the head to each of the aforementioned various positions. Through this, as described above, transient characteristics can be eliminated and a more accurate blood flow velocity after a change in the position of the head can be measured. The predetermined period of time can be, for example, from one to 60 seconds; particularly, from three to 40 seconds; and more particularly, from five to 30 seconds.

Alternatively, the position of the head may be changed so slowly that the transient characteristics can be ignored. The blood flow velocity may be measured after this slow change in the position of the head. If the position of the head is changed slowly in this manner, the movement of the head need not be stopped at a specific position.

h in Formula (9) can be an amount of change in the height of the detection unit 101. h may be measured by the position change amount measurement unit 105. The position change amount measurement unit 105 may be disposed at the center of the front of the head 211 so that the position thereof changes in the same manner as the position of the detection unit 101. For example, by fixing the positional relationship between the position change amount measurement unit 105 and the detection unit 101 within the biological information obtainment device 100, the detection unit 101 and the position change amount measurement unit 105 undergo the same changes in position. This makes it possible to regard the amount of change in the position of the position change amount measurement unit 105 as the amount of change in the position of the detection unit 101.

h in Formula (9) may be measured by an accelerometer, a gyrosensor, an atmospheric pressure sensor, or the like, for example. In other words, the position change amount measurement unit 105 can include, for example, an accelerometer, a gyrosensor, an atmospheric pressure sensor, or the like. The amount of change h in the height in Formula (9) may be determined based on changes in the atmospheric pressure determined by the atmospheric pressure sensor.

For example, the detection unit 101 measures the amount of fluctuation in the blood flow velocity, and the position change amount measurement unit 105 measures the amount of change in the position, in each of cases where the head is at various heights. These measurements may be taken multiple times at each height. The amount of fluctuation in the blood flow velocity and the amount of change in the position at each height are plotted on a vertical axis and a horizontal axis, respectively. The slope of a line obtained by applying the least-squares method to the plotted data may be employed as the position characteristic correction parameter $\alpha$.

Like $\tau$ in Formula (7), $\tau$ in Formula (9) is called a transient characteristic correction parameter. The transient characteristic correction parameter is a parameter pertaining to the transient characteristics of the blood flow velocity. As described above, changes in the blood flow velocity caused by changes in the position of the head have transient characteristics. The transient characteristic correction parameter can be used to reflect these transient characteristics in the measurement value of the blood flow velocity.

The transient characteristic correction parameter $\tau$ may be calculated, for example, by fitting a blood flow velocity estimated value estimated using the position characteristic correction parameter $\alpha$ to transient blood flow velocity data measured over time as the position of the detection unit 101 changes from the reference position to another position. The fitting may be performed through linear fitting or non-linear fitting, for example. The correction parameter setting unit 109 may calculate the transient characteristic correction parameter as described above and set that parameter, for example.

[(2-3) Identifying Pseudo-Blood Flow Velocity Component]

As described above, according to one embodiment of the present technique, using the correction information generated by the correction information generation unit 107, the correction unit 108 corrects the blood flow velocity included in the hemodynamic signal detected by the detection unit 101. This correction information can be calculated using the position characteristic correction parameter $\alpha$ and the transient characteristic correction parameter $\tau$, as indicated by the foregoing Formula (7) and Formula (9). The position characteristic correction parameter $\alpha$ and the transient characteristic correction parameter $\tau$ are then set based on blood flow velocity characteristics included in the hemodynamic signal.

However, as described above, a hemodynamic signal detected from an organism is a composite of component signals caused by a plurality of factors. As such, the blood flow velocity characteristics may be affected by component signals which are not pseudo-blood flow component signals necessary for the calculation of the position characteristic correction parameter $\alpha$ and the transient characteristic correction parameter $\tau$. When affected by component signals which are not pseudo-blood flow component signals, there is a risk that the position characteristic correction parameter $\alpha$ and the transient characteristic correction parameter $\tau$ cannot be correctly calculated and set.

Accordingly, the signal decomposition unit 102 decomposes the hemodynamic signal including blood flow velocity, corrected by the correction unit 108, into a plurality of component signals. The techniques described in the other embodiments can be used for this decomposition.

Then, the identification unit 104 identifies the pseudo-blood flow component signal from the plurality of component signals. Specifically, the identification unit 104 identifies the pseudo-blood flow component signal from the plurality of component signals based on the correlation between the component signals obtained by the decomposition performed by the signal decomposition unit 102 and the correction information generated by the correction information generation unit 107. The pseudo-blood flow component signal is highly correlated with the correction information. Component signals which are not the pseudo-blood flow component signal have low correlation with the correction information. Using these characteristics, the identification unit 104 identifies a pseudo-blood flow component signal from the plurality of component signals.

As described in the second embodiment, the detection unit 101 may include at least two light receiving units. The signal decomposition unit 102 decomposes a first hemodynamic signal and a second hemodynamic signal, which have been detected from at least two locations of an organism, into a plurality of component signals each. The identification unit 104 identifies hemodynamic elements based on the correlation between the periodicity of the component signals included in the first hemodynamic signal and the periodicity of the component signals included in the second hemodynamic signal.

Through this, the biological information obtainment device 100 can obtain a correlation between hemodynamic signals detected from different blood vessels. In particular, the biological information obtainment device 100 according to the third embodiment of the present technique can obtain the correlation between pseudo-blood flow component signals included in the hemodynamic signal.

The method by which the identification unit 104 evaluates the correlation is not limited, but an example thereof will be described. An evaluation value E of the correlation is calculated through the following Formula (10), using a component signal $D_{Ai}$ included in the first hemodynamic signal $S_A$, a component signal $D_{Bi}$ included in the second hemodynamic signal $S_B$, the fluctuation amount v, and a correlation function CC.

[Math. 10]

$$E = \frac{CC(D_{Ai}, v) \times CC(D_{Bi}, v)}{CC(D_{Ai}, D_{Bi})} \quad \cdots \quad (10)$$

As described above, the component signals (Intrinsic Mode Function (IMF)) obtained from the decomposing performed by the signal decomposition unit 102 using the EMD algorithm tend to be output in order from signals having a higher number of extreme values. Accordingly, the identification unit 104 can calculate the evaluation value E for the correlation according to the foregoing Formula (10), in the order in which the component signals are output. The identification unit 104 can then identify the component signal having the highest evaluation value E for the correlation as the pseudo-blood flow component signal.

[(2-4) Updating Correction Parameters]

As described above, according to one embodiment of the present technique, the correction parameter updating unit 110 updates the position characteristic correction parameter and/or the transient characteristic correction parameter so as to reduce the pseudo-blood flow component signal, which acts as noise.

Processing performed by the correction parameter updating unit 110 will be described with reference to Fig. 15. Fig. 15 is a flowchart illustrating an example of the processing by the correction parameter updating unit 110 according to one embodiment of the present technique.

As illustrated in Fig. 15, in step S11, the correction parameter updating unit 110 evaluates the amplitude of the pseudo-blood flow component signal identified by the identification unit 104.

One cause of the position characteristic correction parameter and/or the transient characteristic correction parameter not being calculated correctly is that the corrected blood flow velocity contains a pseudo-blood flow velocity component. When a pseudo-blood flow velocity component is present, the amplitude of the pseudo-blood flow component signal identified by the identification unit 104 increases. Accordingly, the correct position characteristic correction parameter and/or transient characteristic correction parameter are calculated by the correction parameter updating unit 110 evaluating and reducing this amplitude.

The method by which the correction parameter updating unit 110 evaluates the amplitude of the pseudo-blood flow component signal is not particularly limited, but one example thereof will be given here. An amplitude $Amp_{DAi}$ of the component signal $D_{Ai}$ is calculated according to the following Formula (11).

[Math. 11]

$$Amp_{D_{Ai}} = \sum_{t} \left( D_{Ai}(t) \right)^2 \quad \cdots \quad (11)$$

An amplitude Amp of a signal constituted by the component signal $D_{Ai}$ and the component signal $D_{Bi}$ is calculated through the following Formula (12).

[Math. 12]

$$Amp = Amp_{D_{Ai}} + Amp_{D_{Bi}} \quad \cdots (12)$$

Next, in step S12, the correction parameter updating unit 110 updates the position characteristic correction parameter and/or the transient characteristic correction parameter based on the component signal identified by the identification unit 104 and the correction information generated by the correction information generation unit 107. To give a specific example, an updated position characteristic correction parameter $\alpha'$ and/or transient characteristic correction parameter $\tau'$ are calculated through the following Formula (13), using the component signal $D_{Ai}$, the component signal $D_{Bi}$, the fluctuation amount v, and a channel number N. [Math. 13]

$$\alpha', \quad \tau' = \arg \min_{\alpha', \ \tau'} \left| v + \frac{(D_{Ai} + D_{Bi})}{N} - \alpha' \times \sin\theta e^{-\tau'/t} \right|^2 \quad \cdots (13)$$

The updated position characteristic correction parameter $\alpha'$ and/or transient characteristic correction parameter $\tau'$ are transmitted to the correction information generation unit 107. The correction information generation unit 107 uses the updated position characteristic correction parameter $\alpha'$ and/or transient characteristic correction parameter $\tau'$ to generate the correction information according to the foregoing Formula (7) or Formula (9). Using the correction information generated by the correction information generation unit 107, the correction unit 108 corrects the blood flow velocity included in the hemodynamic signal detected by the detection unit 101. The signal decomposition unit 102 decomposes the hemodynamic signal corrected by the correction unit 108 into a plurality of component signals. The identification unit 104 identifies a pseudo-blood flow component signal from the plurality of component signals. The correction parameter updating unit 110 updates the position characteristic correction parameter and/or the transient characteristic correction parameter so as to reduce the pseudo-blood flow component signal, which acts as noise.

Next, in step S13, the correction parameter updating unit 110 determines whether the amplitude Amp is less than or equal to a threshold. Note that this threshold is not particularly limited.

When the amplitude Amp is not less than or equal to the threshold (step S13: No), the correction parameter updating unit 110 performs the processing of steps S11 to 13. The processing of steps S11 to S13 is repeated until the amplitude Amp is less than or equal to the threshold.

When the amplitude Amp is less than or equal to the threshold (step S13: Yes), the correction parameter updating unit 110 ends the processing.

A highly-accurate blood flow velocity component, from which the pseudo-blood flow velocity component has been removed, is obtained by performing such processing. Additionally, the updated position characteristic correction parameter $\alpha'$ and/or transient characteristic correction parameter $\tau'$ are also highly accurate, and can be used in other processing as well.

[4. Fourth Embodiment of Present Technique (Example 4 of Biological Information Obtainment Device)]

The biological information obtainment device 100 according to a fourth embodiment of the present technique adds the periodicity calculation unit 103 to the constituent elements of the biological information obtainment device 100 according to the third embodiment. This will be described with reference to Fig. 16. Fig. 16 is a block diagram illustrating the configuration of the biological information obtainment device 100 according to the fourth embodiment of the present technique.

As illustrated in Fig. 16, the biological information obtainment device 100 according to the fourth embodiment of the present technique can further include the periodicity calculation unit 103. As described in the first embodiment, the periodicity calculation unit 103 calculates a periodicity of each of the plurality of component signals obtained from the decomposing by the signal decomposition unit 102. Features of the component signals are obtained as a result.

Through this, the identification unit 104 can accurately identify hemodynamic elements based on the periodicity calculated by the periodicity calculation unit 103. In particular, the identification unit 104 can accurately identify the pseudo-blood flow component signal.

[5. Fifth Embodiment of Present Technique (Biological Information Obtainment System)]

The configuration of a biological information obtainment system according to one embodiment of the present technique will be described with reference to Fig. 17. Fig. 17 is a block diagram illustrating the configuration of a biological information obtainment system 1000 according to a fifth embodiment of the present technique. As illustrated in Fig. 17, the biological information obtainment system 1000 according to one embodiment of the present technique can include the detection unit 101, the signal decomposition unit 102, the periodicity calculation unit 103, and the identification unit 104.

The detection unit 101 detects a hemodynamic signal from a human organism. The signal decomposition unit 102 decomposes the hemodynamic signal detected from the organism into a plurality of component signals. The periodicity calculation unit 103 calculates a periodicity of each of the plurality of component signals. The identification unit 104 identifies hemodynamic elements based on the periodicity.

The biological information obtainment system 1000 can use the techniques according to the other embodiments described above. As such, the constituent elements included in the biological information obtainment system 1000 will not be described in detail.

[6. Sixth Embodiment of Present Technique (Biological Information Obtainment Method)]

[(1) Description of Sixth Embodiment]

The present technique provides a biological information obtainment method that includes: decomposing a hemodynamic signal detected from an organism into a plurality of component signals; calculating a periodicity of each of the plurality of component signals; and identifying a hemodynamic element based on the periodicity. Hemodynamic elements present in the hemodynamic signal can be obtained accurately through this biological information obtainment method.

[(2) Example of Sixth Embodiment (Biological Information Obtainment Method)]

A biological information obtainment method according to one embodiment of the present technique will be described with reference to Fig. 18. Fig. 18 is a flowchart illustrating an example of a sequence in the biological information obtainment method according to one embodiment of the present technique.

As illustrated in Fig. 18, the biological information obtainment method according to one embodiment of the present technique includes: decomposing a hemodynamic signal detected from an organism into a plurality of component signals (step S1); calculating a periodicity of each of the plurality of component signals (step S2); and identifying a hemodynamic element based on the periodicity (step S3).

The biological information obtainment method according to the present embodiment may use the techniques according to the other embodiments described above. As such, the techniques described in the above embodiments will not be described again here.

The biological information obtainment method according to the present embodiment can be implemented by using software and hardware. Specifically, the biological information obtainment method according to the present embodiment can be implemented by a CPU provided in the hardware reading a program for implementing the biological information obtainment method according to the present embodiment, for example.

In addition to this, the configurations described in the above embodiments can be selected or changed as appropriate to other configurations as long as doing so does not depart from the essential spirit of the present technique.

The effects described in the present specification are merely illustrative and not limitative, and other effects may be obtained.

[7. Working Example]

A detection unit using the LDF method and a three-axis accelerometer were placed at the center of the forehead of a subject (a human). The positional relationship between the detection unit and the three-axis accelerometer was fixed. The subject first faced forward for five seconds, then faced downward for ten seconds, and then faced forward for another ten seconds. The detection unit continuously measured the blood flow velocity at the forehead, and output values were continuously obtained from the three-axis accelerometer, over this total of 25 seconds. The tilt θ was calculated through the above-described Formula (5) using the output values of the three-axis accelerometer, and this was taken as the tilt of the head of the subject. As illustrated in Fig. 12, the tilt θ is an angle projected onto the plane P passing through the detection unit and the centerline X of the head. Fig. 19 illustrates the relationship between the measured blood flow velocity and the tilt θ. In Fig. 19, the scale on the axis on the left side represents a blood flow velocity S (unit: a.u.); the scale on the axis on the right side represents the tilt θ (unit: degrees); and the horizontal axis represents time t (unit: seconds). In Fig. 19, the dotted line represents the tilt θ of the head, and the solid line represents the measured blood flow velocity.

As indicated in Fig. 19, the blood flow velocity from five to 15 seconds, when the head was facing downward, was higher than the blood flow velocity at other times.

The subject then changed posture in the same manner as described above, facing forward for five seconds, facing downward for ten seconds, and then facing forward for another ten seconds. The blood flow velocity at the forehead was measured, and the tilt θ of the head was calculated, in the same manner as described above. A correction was then made to subtract the amount of fluctuation calculated through the above-described Formula (4) from the measured blood flow velocity. Values calculated in advance were used for α and τ in Formula (4). The relationship between the blood flow velocity and the tilt θ of the head after the correction is indicated in Fig. 20. In Fig. 20, the scale on the vertical axis represents the blood flow velocity S (unit: a.u.), and the horizontal axis represents time t (unit: seconds). In Fig. 20, the solid line represents the blood flow velocity after correction, and the dotted line represents the blood flow velocity before correction.

From Fig. 20, it can be seen that the blood flow velocity based on the head tilt was successfully removed through the correction. Accordingly, the present technique can remove the amount of fluctuation in the blood flow velocity caused by changes in the position of the head from the measured blood flow velocity, and can obtain blood flow velocity data from which the health state and/or psychological state of a human can be more accurately ascertained.

[Reference Signs List]

| | |
|---|---|
| 100 | Biological information obtainment device |
| 101 | Detection unit |
| 111 | Light emitting unit |
| 112 | Light receiving unit |
| 102 | Signal decomposition unit |
| 103 | Periodicity calculation unit |
| 104 | Identification unit |
| 105 | Position change amount measurement unit |
| 106 | Processing unit |
| 107 | Correction information generation unit |
| 108 | Correction unit |
| 109 | Correction parameter setting unit |
| 110 | Correction parameter updating unit |
| 1000 | Biological information obtainment system |
| S1 | Decomposing to component signals |
| S2 | Calculating periodicity |
| S3 | Identifying hemodynamic element |

**Claims**

**1.** A biological information obtainment device (100) comprising:

a signal decomposition unit (102) configured to decompose a hemodynamic signal detected from an organism into a plurality of component signals;
a periodicity calculation unit (103) configured to calculate a periodicity of each of the plurality of component signals; and
an identification unit (104) configured to identify a hemodynamic element based on the periodicity, wherein the signal decomposition unit (102) is configured to decompose a first hemodynamic signal and a second hemodynamic signal detected from at least two locations of the organism into a plurality of component signals each, and the identification unit (104) is configured to identify the hemodynamic element based on a correlation between the periodicity of the component signals included in the first hemodynamic signal and the periodicity of the component signals included in the second hemodynamic signal and an evaluation value E of the correlation calculated through Formula (3) using an evaluation value $P_{A1}$ of the periodicity of a component signal $D_{A1}$ included in a first hemodynamic signal $S_A$, an evaluation value $P_{B1}$ of the periodicity of a component signal $D_{B1}$ included in a second hemodynamic signal $S_B$, and a number N of sampling data contained in the component signals, wherein Formula (3) comprises:

$$E = \sum_{i=1}^{N} \left( P_{A1}(i) - P_{B1}(i) \right) \quad \cdots \quad (3)$$

2. The biological information obtainment device according to claim 1, wherein the hemodynamic element includes a heartbeat component, a vasomotion component, and/or a pseudo-blood flow component.

3. The biological information obtainment device according to claim 1, wherein the signal decomposition unit uses empirical mode decomposition.

4. The biological information obtainment device according to claim 1, wherein the periodicity includes autocorrelation.

5. The biological information obtainment device according to claim 4, wherein an autocorrelation $R(\tau)$ is calculated according to Formula (1) below using a value $v(i)$ of the component signal at time i, a delay time $\tau$, and a number N of sampling data contained in the component signal, wherein Formula (1) comprises:

$$R(\tau) = \frac{1}{N} \sum_{i=1}^{N} v(i) \cdot v(i + \tau) \quad \cdots \quad (1)$$

6. The biological information obtainment device according to claim 1, wherein the identification unit is conf igured to identify a component signal for which the evaluation value E of the correlation is highest as a heartbeat component, and is configured to identify a component signal for which the evaluation value E of the correlation is lowest as a vasomotion component.

7. The biological information obtainment device according to claim 1, further comprising:
a detection unit is conf igured to detect the hemodynamic signal.

8. The biological information obtainment device according to claim 7, wherein the detection unit includes:

a light emitting unit is configured to irradiate the organism with light; and
a light receiving unit is configured to detect light produced as a result of irradiating the organism with the light.

9. The biological information obtainment device according to claim 8, wherein the detection unit includes at least two of the light receiving units.

10. The biological information obtainment device according to claim 1, further comprising:

a detection unit, attached to a head, is conf igured to detect the hemodynamic signal;
a position change amount measurement unit is conf igured to measure an amount of change in a position of the detection unit;
a correction information generation unit is configured to generate correction information for a blood flow velocity based on the amount of change in the position; and
a correction unit that, using the correction information, is configured to correct a blood flow velocity included in the hemodynamic signal detected by the detection unit.

11. The biological information obtainment device according to claim 10,

wherein the amount of change is an amount of change in a tilt of the detection unit or an amount of change in a height of the detection unit.

12. The biological information obtainment device according to claim 10,

wherein the signal decomposition unit is configured to decompose the hemodynamic signal including the blood flow velocity corrected by the correction unit into a plurality of component signals, and
the identification unit is configured to identify the hemodynamic element based on a correlation between the component signals and the correction information.

13. A biological information obtainment method comprising:

decomposing, by a signal decomposition unit (102), a first hemodynamic signal and a second hemodynamic signal detected from at least two locations on an organism into a plurality of component signals;
calculating, by a periodicity calculation unit (103), a periodicity of each of the plurality of component signals; and
identifying, by an identification unit (104), a hemodynamic element based on a correlation between the periodicity of the component signals included in the first hemodynamic signal and the periodicity of the component signals included in the second hemodynamic signal and an evaluation value E of the correlation calculated through Formula (3) using an evaluation value $P_{A1}$ of the periodicity of a component signal $D_{A1}$ included in a first hemodynamic signal $S_A$, an evaluation value $P_{B1}$ of the periodicity of a component signal $D_{B1}$ included in a second hemodynamic signal $S_B$, and a number N of sampling data contained in the component signals, wherein the Formula (3) comprises:

$$E = \sum_{i=1}^{N} \left( P_{A1}(i) - P_{B1}(i) \right) \quad \cdots \quad (3)$$

**Patentansprüche**

1. Erlangungsvorrichtung für biologische Informationen (100), umfassend:

eine Signalzerlegungseinheit (102), die konfiguriert ist, um ein hämodynamisches Signal, das von einem Organismus erfasst wird, in eine Vielzahl von Komponentensignalen zu zerlegen;
eine Periodizitätsberechnungseinheit (103), die konfiguriert ist, um eine Periodizität jedes der Vielzahl von Komponentensignalen zu berechnen; und
eine Identifikationseinheit (104), die konfiguriert ist, um ein hämodynamisches Element basierend auf der Periodizität zu identifizieren,
wobei die Signalzerlegungseinheit (102) konfiguriert ist, um ein erstes hämodynamisches Signal und ein zweites hämodynamisches Signal, die von mindestens zwei Stellen des Organismus erfasst werden, in jeweils eine Vielzahl von Komponentensignalen zu zerlegen, und
die Identifikationseinheit (104) konfiguriert ist, um das hämodynamische Element basierend auf einer Korrelation zwischen der Periodizität der Komponentensignale, die in dem ersten hämodynamischen Signal eingeschlossen sind, und der Periodizität der Komponentensignale, die in dem zweiten hämodynamischen Signal eingeschlossen sind, und einem Bewertungswert E der Korrelation zu identifizieren, der über Formel (3) unter Verwendung eines Bewertungswerts $P_{A1}$ der Periodizität eines Komponentensignals $D_{A1}$, das in einem ersten hämodynamischen Signal $S_A$ eingeschlossen ist, eines Bewertungswerts $P_{B1}$ der Periodizität eines Komponentensignals $D_{B1}$, das in einem zweiten hämodynamischen Signal $S_B$ eingeschlossen ist, und einer Anzahl N von Abtastdaten, die in den Komponentensignalen enthalten sind, berechnet wird, wobei Formel (3) umfasst:

$$E = \sum_{i=1}^{N} \left( P_{A1}(i) - P_{B1}(i) \right) \quad \cdots \quad (3)$$

**2.** Erlangungsvorrichtung für biologische Informationen nach Anspruch 1,
wobei das hämodynamische Element eine Herzschlagkomponente, eine Vasomotionskomponente und/oder eine Pseudoblutflusskomponente umfasst.

**3.** Erlangungsvorrichtung für biologische Informationen nach Anspruch 1,
wobei die Signalzerlegungseinheit eine empirische Modenzerlegung verwendet.

**4.** Erlangungsvorrichtung für biologische Informationen nach Anspruch 1,
wobei die Periodizität Autokorrelation einschließt.

**5.** Erlangungsvorrichtung für biologische Informationen nach Anspruch 4,
wobei eine Autokorrelation $R(\tau)$ gemäß nachstehender Formel (1) unter Verwendung eines Werts $v(i)$ des Komponentensignals zu einem Zeitpunkt $i$, einer Verzögerungszeit $\tau$ und einer Anzahl N von Abtastdaten, die in dem Komponentensignal enthalten sind, berechnet wird, wobei Formel (1) umfasst:

$$R(\tau) = \frac{1}{N} \sum_{i=1}^{N} v(i) \cdot v(i + \tau) \quad \cdots \quad (1)$$

**6.** Erlangungsvorrichtung für biologische Informationen nach Anspruch 1,
wobei die Identifikationseinheit konfiguriert ist, um ein Komponentensignal, für das der Bewertungswert E der Korrelation am höchsten ist, als eine Herzschlagkomponente zu identifizieren, und konfiguriert ist, um ein Komponentensignal, für das der Bewertungswert E der Korrelation am niedrigsten ist, als eine Vasomotionskomponente zu identifizieren.

**7.** Erlangungsvorrichtung für biologische Informationen nach Anspruch 1, ferner umfassend:
eine Erfassungseinheit ist konfiguriert, um das hämodynamische Signal zu erfassen.

**8.** Erlangungsvorrichtung für biologische Informationen nach Anspruch 7,
wobei die Erfassungseinheit einschließt:

eine Lichtemissionseinheit ist konfiguriert, um den Organismus mit Licht zu bestrahlen; und
eine Lichtempfangseinheit ist konfiguriert, um Licht zu erfassen, das als Ergebnis eines Bestrahlens des Organismus mit dem Licht produziert wird.

**9.** Erlangungsvorrichtung für biologische Informationen nach Anspruch 8, wobei die Erfassungseinheit mindestens zwei der Lichtempfangseinheiten einschließt.

**10.** Erlangungsvorrichtung für biologische Informationen nach Anspruch 1, ferner umfassend:

eine Erfassungseinheit, die an einem Kopf angebracht ist, ist konfiguriert, um das hämodynamische Signal zu erfassen;
eine Positionsänderungsbetragsmessungseinheit ist konfiguriert, um einen Betrag einer Änderung einer Position der Erfassungseinheit zu messen;
eine Korrekturinformationserzeugungseinheit ist konfiguriert, um Korrekturinformationen für eine Blutflussgeschwindigkeit basierend auf dem Betrag der Änderung der Position zu erzeugen; und
eine Korrektureinheit, die, unter Verwendung der Korrekturinformationen, konfiguriert ist, um eine Blutflussgeschwindigkeit zu korrigieren, die in dem hämodynamischen Signal eingeschlossen ist, das durch die Erfassungseinheit erfasst wird.

**11.** Erlangungsvorrichtung für biologische Informationen nach Anspruch 10,
wobei der Betrag der Änderung ein Betrag der Änderung einer Neigung der Erfassungseinheit oder ein Betrag der Änderung einer Höhe der Erfassungseinheit ist.

**12.** Erlangungsvorrichtung für biologische Informationen nach Anspruch 10,

wobei die Signalzerlegungseinheit konfiguriert ist, um das hämodynamische Signal einschließlich der Blutflussgeschwindigkeit, die durch die Korrektureinheit korrigiert wird, in eine Vielzahl von Komponentensignalen zu zerlegen, und

die Identifikationseinheit konfiguriert ist, um das hämodynamische Element basierend auf einer Korrelation zwischen den Komponentensignalen und den Korrekturinformationen zu identifizieren.

**13.** Erlangungsverfahren für biologische Informationen, umfassend:

Zerlegen, durch eine Signalzerlegungseinheit (102), eines ersten hämodynamischen Signals und eines zweiten hämodynamischen Signals, die von mindestens zwei Stellen an einem Organismus erfasst werden, in eine Vielzahl von Komponentensignalen;
Berechnen, durch eine Periodizitätsberechnungseinheit (103), einer Periodizität jedes der Vielzahl von Komponentensignalen; und
Identifizieren, durch eine Identifikationseinheit (104),
eines hämodynamischen Elements basierend auf einer Korrelation zwischen der Periodizität der Komponentensignale, die in dem ersten hämodynamischen Signal eingeschlossen sind, und der Periodizität der Komponentensignale, die in dem zweiten hämodynamischen Signal eingeschlossen sind, und einem Bewertungswert E der Korrelation, der über Formel (3) unter Verwendung eines Bewertungswerts $P_{A1}$ der Periodizität eines Komponentensignals $D_{A1}$, das in einem ersten hämodynamischen Signal $S_A$ eingeschlossen ist, einem Bewertungswert $P_{B1}$ der Periodizität eines Komponentensignals $D_{B1}$, das in einem zweiten hämodynamischen Signal $S_B$ eingeschlossen ist, und einer Anzahl N von Abtastdaten, die in den Komponentensignalen enthalten sind, berechnet wird, wobei die Formel (3) umfasst:

$$E = \sum_{i=1}^{N} \left( P_{A1}(i) - P_{B1}(i) \right) \quad \cdots \quad (3)$$

**Revendications**

**1.** Dispositif d'obtention d'informations biologiques (100) comprenant :

une unité de décomposition de signal (102) configurée pour décomposer un signal hémodynamique détecté à partir d'un organisme en une pluralité de signaux constitutifs ;
une unité de calcul de périodicité (103) configurée pour calculer une périodicité de chacun de la pluralité de signaux constitutifs ; et
une unité d'identification (104) configurée pour identifier un élément hémodynamique sur la base de la périodicité, dans lequel l'unité de décomposition de signal (102) est configurée pour décomposer un premier signal hémodynamique
et un second signal hémodynamique détectés à partir d'au moins deux emplacements de l'organisme en une pluralité de signaux constitutifs chacun, et
l'unité d'identification (104) est configurée pour identifier l'élément hémodynamique sur la base d'une corrélation entre la périodicité des signaux constitutifs inclus dans le premier signal hémodynamique et la périodicité des signaux constitutifs inclus dans le second signal hémodynamique et une valeur d'évaluation E de la corrélation calculée par la Formule (3) à l'aide d'une valeur d'évaluation $P_{A1}$ de la périodicité d'un signal constitutif $D_{A1}$ inclus dans un premier signal hémodynamique $S_A$, d'une valeur d'évaluation $P_{B1}$ de la périodicité d'un signal constitutif $D_{B1}$ inclus dans un second signal hémodynamique $S_B$, et d'un nombre N de données d'échantillonnage contenues dans les signaux constitutifs, dans lequel la Formule (3) comprend :

$$E = \sum_{i=1}^{N} \left( P_{A1}(i) - P_{B1}(i) \right) \quad \cdots \quad (3)$$

**2.** Dispositif d'obtention d'informations biologiques selon la revendication 1,
dans lequel l'élément hémodynamique comporte une composante de battement cardiaque, une composante de vasomotricité, et/ou une composante de pseudo-flux sanguin.

**3.** Dispositif d'obtention d'informations biologiques selon la revendication 1,
dans lequel l'unité de décomposition de signal utilise une décomposition en mode empirique.

**4.** Dispositif d'obtention d'informations biologiques selon la revendication 1,
dans lequel la périodicité comporte une autocorrélation.

**5.** Dispositif d'obtention d'informations biologiques selon la revendication 4,
dans lequel une autocorrélation R($\tau$) est calculée selon la Formule (1) ci-après à l'aide d'une valeur v(i) du signal constitutif au temps i, d'un temps de retard $\tau$, et d'un nombre N de données d'échantillonnage contenues dans le signal constitutif, dans lequel la Formule (1) comprend :

$$R(\tau) = \frac{1}{N} \sum_{i=1}^{N} v(i) \cdot v(i + \tau) \quad \cdots \quad (1)$$

**6.** Dispositif d'obtention d'informations biologiques selon la revendication 1,
dans lequel l'unité d'identification est configurée pour identifier un signal constitutif pour lequel la valeur d'évaluation E de la corrélation est la plus élevée en tant que composante de battement cardiaque, et est configurée pour identifier un signal constitutif pour lequel la valeur d'évaluation E de la corrélation est la plus faible en tant que composante de vasomotricité.

**7.** Dispositif d'obtention d'informations biologiques selon la revendication 1, comprenant en outre :
une unité de détection est configurée pour détecter le signal hémodynamique.

**8.** Dispositif d'obtention d'informations biologiques selon la revendication 7,
dans lequel l'unité de détection comporte :

une unité d'émission de lumière est configurée pour irradier l'organisme avec de la lumière ; et
une unité de réception de lumière est configurée pour détecter la lumière produite à la suite de l'irradiation de l'organisme avec la lumière.

**9.** Dispositif d'obtention d'informations biologiques selon la revendication 8, dans lequel l'unité de détection comporte au moins deux des unités de réception de lumière.

**10.** Dispositif d'obtention d'informations biologiques selon la revendication 1, comprenant en outre :

une unité de détection, fixée à une tête, est configurée pour détecter le signal hémodynamique ;
une unité de mesure d'amplitude de changement de position est configurée pour mesurer une amplitude de changement de position de l'unité de détection ;
une unité de génération d'informations de correction est configurée pour générer des informations de correction pour une vitesse d'écoulement sanguin sur la base de l'amplitude de changement de position ; et
une unité de correction qui, à l'aide des informations de correction, est configurée pour corriger une vitesse d'écoulement sanguin incluse dans le signal hémodynamique détecté par l'unité de détection.

**11.** Dispositif d'obtention d'informations biologiques selon la revendication 10,

dans lequel l'amplitude de changement est une amplitude de changement d'une inclinaison de l'unité de détection ou une amplitude de changement d'une hauteur de l'unité de détection.

**12.** Dispositif d'obtention d'informations biologiques selon la revendication 10,

dans lequel l'unité de décomposition de signal est configurée pour décomposer le signal hémodynamique, y compris la vitesse de flux sanguin corrigée par l'unité de correction, en une pluralité de signaux constitutifs, et l'unité d'identification est configurée pour identifier l'élément hémodynamique sur la base d'une corrélation entre les signaux constitutifs et les informations de correction.

**13.** Procédé d'obtention d'informations biologiques comprenant :

la décomposition, par une unité de décomposition de signal (102), d'un premier signal hémodynamique et d'un second signal hémodynamique détectés à partir d'au moins deux emplacements d'un organisme en une pluralité de signaux constitutifs ;
le calcul, par une unité de calcul de périodicité (103), d'une périodicité de chacun de la pluralité de signaux constitutifs ; et
l'identification, par une unité d'identification (104),
d'un élément hémodynamique sur la base d'une corrélation entre la périodicité des signaux constitutifs inclus dans le premier signal hémodynamique et la périodicité des signaux constitutifs inclus dans le second signal hémodynamique et une valeur d'évaluation E de la corrélation calculée par la Formule (3) à l'aide d'une valeur d'évaluation $P_{A1}$ de la périodicité d'un signal constitutif $D_{A1}$ inclus dans un premier signal hémodynamique $S_A$, d'une valeur d'évaluation $P_{B1}$ de la périodicité d'un signal constitutif $D_{B1}$ inclus dans un second signal hémodynamique $S_B$, et d'un nombre N de données d'échantillonnage contenues dans les signaux constitutifs, dans lequel la Formule (3) comprend :

$$\mathrm{E} = \sum_{i=1}^{N} \big( P_{A1}(i) - P_{B1}(i) \big) \quad \cdots \quad (3)$$

Fig. 1

HEMODYNAMIC SIGNAL $S_A$

HEARTBEAT COMPONENT $D_{A1}$

VASOMOTION COMPONENT $D_{A2}$

OTHER $D_{A3}$

Fig. 2

Fig. 3

HEARTBEAT COMPONENT

$W_1$

A

$D_{A1}$

VASOMOTION COMPONENT

$W_2$   $D_{A2}$

B

Fig. 4

100

301 CPU

302 STORAGE

303 RAM

304 OPERATION UNIT

305 DISPLAY UNIT

Fig. 5

Fig. 6

Fig. 7

HEARTBEAT
COMPONENT

A

CHANNEL A $D_{A1}$

CHANNEL B $D_{B1}$

150  160  170  180  190  200  210  220  230  240  250

TIME [s]

VASOMOTION
COMPONENT

H      L

B

CHANNEL A $D_{A2}$

CHANNEL B $D_{B2}$

150  160  170  180  190  200  210  220  230  240  250

TIME [s]

34

Fig. 8

HEARTBEAT
COMPONENT

CHANNEL A — $W_{A1}$ / $D_{A1}$

CHANNEL B — $W_{B1}$ / $D_{B1}$

VASOMOTION
COMPONENT

CHANNEL A — $D_{A2}$ / $W_{A2}$

CHANNEL B — $D_{B2}$ / $W_{B2}$

A

B

Fig. 9

Fig. 10

Fig. 11

Fig. 12

Fig. 13

Fig. 14

Fig. 15

```
                    ┌──────────┐
                    │  START   │
                    └────┬─────┘
                         │
                         ▼
        ┌────────────────────────────────────────┐  ⌇ S11
        │  EVALUATE AMPLITUDE OF PSEUDO-BLOOD     │
        │       FLOW COMPONENT SIGNAL             │
        └────────────────┬───────────────────────┘
                         │                           ⌇ S12
        ┌────────────────▼───────────────────────┐
        │       UPDATE CORRECTION PARAMETERS      │
        └────────────────┬───────────────────────┘
                         │                           ⌇ S13
    No     ╲─────────────▼──────────────╱
        ╲    AMPLITUDE LESS THAN OR EQUAL TO  ╱
          ╲          THRESHOLD?             ╱
                         │
                        Yes
                         ▼
                    ┌──────────┐
                    │   END    │
                    └──────────┘
```

Fig. 16

Fig. 17

| DETECTION UNIT | SIGNAL DECOMPOSITION UNIT | PERIODICITY CALCULATION UNIT | IDENTIFICATION UNIT |

1000
101
102
103
104

$S_A$
$D_{A1}$ $D_{A2}$
$P_{A1}$ $P_{A2}$

$S_B$
$D_{B1}$ $D_{B2}$
$P_{B1}$ $P_{B2}$

Fig. 18

```
        ┌─────────────┐
        │    START    │
        └──────┬──────┘
               │
               ▼
┌──────────────────────────────────┐  ⌇S1
│   DECOMPOSE INTO PLURALITY OF     │
│      COMPONENT SIGNALS            │
└──────────────┬───────────────────┘
               │
               ▼
┌──────────────────────────────────┐  ⌇S2
│      CALCULATE PERIODICITY        │
└──────────────┬───────────────────┘
               │
               ▼
┌──────────────────────────────────┐  ⌇S3
│   IDENTIFY HEMODYNAMIC ELEMENTS   │
└──────────────┬───────────────────┘
               │
               ▼
        ┌─────────────┐
        │     END     │
        └─────────────┘
```

Fig. 19

Fig. 20

**EP 4 226 852 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017519548 PCT
- JP 2012130391 A
- US 2011169978 A